(19) 

Europäisches
Patentamt
European
Patent Office
Office européen
des brevets

(11) **EP 2 589 330 A1**

(12) **EUROPEAN PATENT APPLICATION**
published in accordance with Art. 153(4) EPC

(43) Date of publication:
**08.05.2013 Bulletin 2013/19**

(21) Application number: **11800253.4**

(22) Date of filing: **17.06.2011**

(51) Int Cl.:
**A61B 5/00** (2006.01)  **A61B 5/11** (2006.01)

(86) International application number:
**PCT/IB2011/001360**

(87) International publication number:
**WO 2012/001473 (05.01.2012 Gazette 2012/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.02.2011 JP 2011035378
30.06.2010 JP 2010149733**

(71) Applicant: **Panasonic Corporation
Kadoma-shi
Osaka 571-8501 (JP)**

(72) Inventors:
• **FUJII, Hiroyuki
  Osaka 540-6207 (JP)**
• **NAKAMOTO, Eiji
  Osaka 540-6207 (JP)**

(74) Representative: **Müller-Boré & Partner
Patentanwälte
Grafinger Straße 2
81671 München (DE)**

(54) **MONITORING DEVICE, AND PROGRAM**

(57)     A monitoring device includes a range imaging sensor, a bed recognition unit, a person recognition unit, a movement determination unit and a notification unit. The range imaging sensor generates a range image wherein pixel values are distance values to an object, the entirety of a bed to be monitored being included in a field of vision thereof. The bed recognition unit extracts a location of the bed using the range image. The person recognition unit detects regions occupied by a person inside and outside of the range of the bed recognized by the bed recognition unit in the range image. The movement determination unit determines the movement of the person relative to the bed using a combination of the regions of the bed detected by the bed recognition unit and the person detected by the person recognition unit. The notification unit notifies a determination result of the movement determination unit.

*FIG. 1*

EP 2 589 330 A1

**Description**

Field of the Invention

The present invention relates to a monitoring device for monitoring a person on a bed, and a program for implementing functions used in the monitoring device in a computer.

Background of the Invention

As such a monitoring device, there has been suggested a technique of obtaining a distance related value corresponding to a distance to an object to be monitored and comparing the distance related value with a threshold value to detect movement or breath of a sleeper on a bed (see, e.g., Japanese Patent Application Publication No. 2003-290154). Described in Japanese Patent Application Publication No. 2003-290154 is a technique, in which when an image change of a sleeper is not detected for a predetermined time, the object to be monitored is determined to be out of a region to be monitored, thereby detecting the object leaving the bed.

In Japanese Patent Application Publication No. 2003-290154, object points including a portion of the sleeper on the bed are set up, and distances to the object points are measured from information of the sleeper by a range sensor. The object points are arranged within a region to be monitored, which is set up to include a portion of a sleeper when the sleeper is on the bed. In the technique described in Japanese Patent Application Publication No. 2003-290154, sleeper's breath or sitting as well as sleeper's leaving the bed is detected by using locations of the object points and a temporal change of distance related values that can be obtained from the object points.

In the technique described in Japanese Patent Application Publication No. 2003-290154, for example, whether the sleeper has left a bed can be detected in a noncontact manner, there is an advantage in that a patient is not stressed. Further, Japanese Patent Application Publication No. 2003-290154 suggests that since the range sensor can be provided in a movable stand, the range sensor may be installed at a desired location if necessary.

However, although Japanese Patent Application Publication No. 2003-290154 discloses that the region to be monitored is installed over the bed, it is not specifically described how to set up the region to be monitored. That is, it is impossible to automatically set up the region to be monitored on the bed. In particular, when the range sensor is movable using the stand, it is not easy to adjust the range sensor to properly set up the object points.

Summary of the Invention

In view of the above, it is an object of the present invention to provide a monitoring device capable of accurately and reproducibly detecting movement of a person to be monitored by automatically detecting a region to be monitored using a bed as a reference, and a program for implementing functions of the monitoring device by a computer.

In accordance with an aspect of the present invention, there is provided a monitoring device, which includes a range imaging sensor for generating a range image wherein pixel values are distance values to an object, the entirety of a bed to be monitored being included in a field of vision of the range imaging sensor; a bed recognition unit for extracting a location of the bed using the range image; a person recognition unit for detecting regions occupied by a person inside and outside of the range of the bed recognized by the bed recognition unit in the range image; a movement determination unit for determining a movement of the person relative to the bed using a combination of the regions of the bed detected by the bed recognition unit and the person detected by the person recognition unit; and a notification unit for notifying a determination result of the movement determination unit.

The bed recognition unit may obtain frequency distributions of heights greater than or equal to a specified height from a floor surface, on which the bed is placed, in lengthwise and widthwise directions of the bed, and locations in which a frequency crosses a threshold are detected as locations of ends of the bed in the lengthwise and widthwise directions.

In such a case, the bed recognition unit may set up a rectangular range surrounded by the ends of the bed in the lengthwise and widthwise directions as the range of the bed, segment the range of the bed into segmented regions in the lengthwise direction of the bed, obtain representative values of heights for respective segmented regions, and use values obtained by subtracting a predetermined offset value from the representative values as reference heights for the respective segmented regions of the bed.

The bed recognition unit may use one of an average value and a mode value of the heights for the segmented regions as the representative value of each of the segmented regions.

The bed recognition unit may provide an upper limit for the representative values of the segmented regions and does not employ the representative values exceeding the upper limit.

The bed recognition unit may provide a threshold height value for the representative values of the segmented regions and determines that the location of the bed is abnormal when representative values of a predetermined number or more of the segmented regions exceed the threshold height value.

Immediately after the location of the bed is extracted, the bed recognition unit may memorize an existence range of an object located in the range of the bed and above a predefined exclusion threshold value for the reference heights of the segmented regions as a mask region excluded from the region of the person detected by the person recognition unit.

The person recognition unit may set up at least one detection plane spaced by a predetermined distance upwardly from the reference heights of the respective segmented regions detected by the bed recognition unit, and determines that the movement of the person is in the range of the bed defined for the detection plane if the object crossing the detection plane is detected.

The person recognition unit may set up a fallen threshold value of a predetermined height from the floor, on which the bed is placed, outside of the range of the bed detected by the bed recognition unit, and determine that the person exists outside of the range of the bed if the object is detected in a location higher than the fallen threshold value outside of the range of the bed.

The person recognition unit may memorize a background image placed outside of the range of the bed detected by the bed recognition unit, and determine that the person exists outside of the range of the bed when an area of a region in which the background image is changed in an image obtained after the memorization is a predefined threshold value.

In such a case, the detection plane may include a bed-ridden state detection plane for detecting a bed-ridden state of the person on the bed and a sitting detection plane for detecting a sitting state of the person on the bed, and the person recognition unit calculates a ratio of an area where the person exists in the sitting detection plane to an area where the person exists in the bed-ridden state detection plane when the person crosses the bed-ridden state detection plane and the sitting detection plane and determines that the person is in a sitting state if the ratio is greater than or equal to a predetermined threshold value.

The detection plane may include a bed-ridden state detection plane for detecting a bed-ridden state of the person on the bed and a stand-up detection plane for detecting a rising (stand-up) state of the person on the bed, and the person recognition unit calculates a ratio of an area cut by the stand-up state detection plane to an area cut by the bed-ridden state detection plane when the person crosses the bed-ridden state detection plane and the stand-up state detection plane and determines that the person is in a stand-up state if the ratio is greater than or equal to a predetermined threshold value.

When a region occupied by the person crosses an end of the bed in the lengthwise direction and widthwise direction, the movement determination unit may determine that the person perches on the bed.

If a region occupied by the person on an end of the bed in the lengthwise direction and widthwise direction spans regions inside and outside of the range of the bed, and a ratio of an area of the region occupied by the person outside of the range of the bed to an area of the region occupied by the person is within a predefined reference range, the movement determination unit may determine that the person perches on the bed.

When the bed is provided with a fence for preventing a fall and a location of the fence can be specified in advance, if a region occupied by the person crosses the fence, the movement determination unit may determine that the person climbs over the fence.

When the bed is provided with a fence for preventing a fall and a location of the fence can be specified in advance, if a region occupied by the person spans regions inside and outside of the range of the bed at the fence and a ratio of an area of a region occupied by the person outside of the range of the bed to an area of the region occupied by the person exceeds a predefined reference range, the movement determination unit may determine that the person climbs over the fence.

When a ratio of an area of a region occupied by the person outside of the range of the bed to an area of the regions occupied by the person inside and outside of the range of the bed exceeds a predefined reference range, the movement determination unit may determine that the person leaves the bed.

When an object having a height less than or equal to a predefined height with respect to the floor surface, on which the bed is placed, outside of the range of the bed is detected continuously for a predefined determination time, the movement determination unit may determine that the person falls down.

The movement determination unit may trace a location of a representative point of the person detected inside and outside of the range of the bed, and the movement determination unit does not determines that the person moves when the location moves discontinuously.

When the location of the representative point of the person is traced, the movement determination unit determines that the person behaves if a starting point is inside of the range of the bed, and updates the starting point of tracing inside of the range of the bed if the starting point is outside of the range of the bed and the person is inside of the range of the bed continuously for a predefined time after the person has moved into the range of the bed.

The notification unit may receive an input from an operating device operated by a person in addition to the determination result of the movement determination unit, and combine the determination result of the movement determination unit and the input by the operating device to switch between existence and non-existence of a notification of the determination result of the movement determination unit.

The range imaging sensor may have a function of converting a coordinate system defined for the range image into a coordinate system of a space having the bed placed therein.

The notification unit may have a function of allowing a notification to correspond with the movement of the person.

The notification unit may be provided with a communication function of notifying a separately provided reception device of a notification.

The range imaging sensor may have a function of generating a gray scale image together with the range image, and the notification unit outputs at least one of the range image and the gray scale image after notifying the movement of the person.

The movement determination unit may have a specific movement detection part for detecting a specific movement of the person; and the notification unit has an image memory part for memorizing at least one of the gray scale image and the range image generated by the range imaging sensor, and a communication processing part for transmitting the gray scale images for a predetermined duration before and after the specific movement detection part detects the specific movement of the person from the image memory part and the range imaging sensor to another device.

The movement determination unit may have a specific movement detection part for detecting a specific movement of the person; and the notification unit has an image memory part for memorizing at least one of the gray scale image and the range image generated by the range imaging sensor, and an observation image memory part for memorizing at least one of the range images and the gray scale images for a predetermined duration before and after the specific movement detection part detects the specific movement of the person.

Alternatively, the movement determination unit may have a specific movement detection part for detecting a specific movement of the person; and the notification unit has an image memory part for memorizing at least one of the gray scale image and the range image generated by the range imaging sensor, an observation image memory part for memorizing at least one of the range images and the gray scale images for a predetermined duration before and after the specific movement detection part detects the specific movement of the person, and a communication processing part for notifying the other device that the specific movement detection part detects the specific movement of the person.

In accordance with another aspect of the present invention, there is provided a program for allowing a computer to function as a bed recognition unit for extracting a location of a bed to be monitored using a range image obtained from a range imaging sensor, the range imaging sensor generating the range image wherein pixel values are distance values to an object, the entirety of the bed being included in a field of vision of the range imaging sensor; a person recognition unit for detecting regions occupied by a person inside and outside of the range of the bed recognized by the bed recognition unit in the range image; a movement determination unit for determining a movement of the person relative to the bed using a combination of the regions of the bed detected by the bed recognition unit and the person detected by the person recognition unit; and a notification unit for notifying a determination result of the movement determination unit.

According to the present invention, since a region to be detected on a bed is automatically detected, it is possible to divide an inside of a range of the bed and an outside thereof from each other and to accurately and reproducibly detect the movement of the person to be monitored.

Brief Description of the Drawings

Fig. 1 is a block diagram showing an entire configuration of a monitoring device according to one embodiment of the present invention;
Fig. 2A is a perspective view showing an exemplary use of the monitoring device, and Fig. 2B is a perspective view showing an example of the monitoring device;
Fig. 3 is a block diagram showing a range imaging sensor used in the monitoring device;
Fig. 4 is a view illustrating a principle of coordinate transformation in the monitoring device;
Figs. 5A to 5C are views illustrating a principle of coordinate transformation in the monitoring device;
Fig. 6 is a perspective view showing another exemplary use of the monitoring device;
Figs. 7A and 7B are views illustrating the operation of detecting ends of a bed in the monitoring device;
Fig. 8 is a view illustrating the operation of the monitoring device when a field of vision is not suitable;
Fig. 9A is a view illustrating segmented regions of the monitoring device, and Fig. 9B is a view illustrating a bed surface of the monitoring device;
Fig. 10 is a view illustrating the operation of the monitoring device;
Fig. 11 is a view illustrating the operation of the monitoring device;
Fig. 12 is a view illustrating the operation of the monitoring device;

Fig. 13 is a view illustrating the operation of the monitoring device;

Fig. 14 is a perspective view showing an example in which an object overlaps the bed when using the monitoring device;

Figs. 15A and 15B are views illustrating the exemplary operation of the monitoring device in which a mask region is set up;

Fig. 16A is a plan view showing an example in which an object overlaps the bed when using the monitoring device, and Fig. 16B is a side view thereof;

Fig. 17 is a view showing an example of the monitoring device in which a detection plane is set up;

Figs. 18A and 18B are views illustrating the operation of the monitoring device;

Figs. 19A and 19B are views illustrating the operation of the monitoring device;

Figs. 20A to 20C are views illustrating the operation of the monitoring device;

Figs. 21A to 21C are views illustrating the operation of the monitoring device;

Fig. 22 is a view illustrating the operation of the monitoring device;

Fig. 23 is a block diagram showing another configuration of the monitoring device;

Fig. 24 is a block diagram showing still another configuration of the monitoring device;

Fig. 25 is a block diagram showing another configuration of the monitoring device; and

Fig. 26 is a perspective view showing an example of the monitoring device in which a bedside terminal is arranged.

Detailed Description of the Embodiments

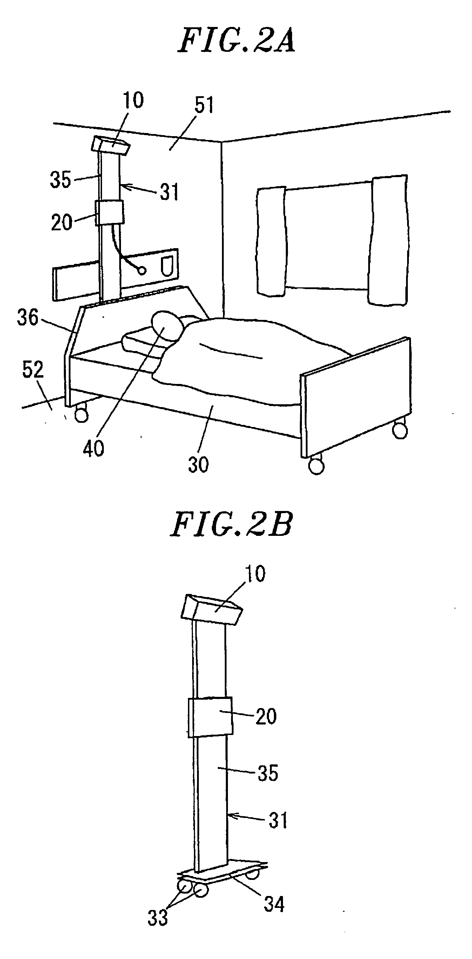

Hereinafter, the entire configuration of a monitoring device will be described. In the monitoring device of this embodiment, a range imaging sensor 10 is used to take a range image, wherein the entirety of a bed 30 is included in a field of vision of the range imaging sensor, as shown in Figs. 2A and 2B. The range image is an image having pixel values being distance values. A variety of techniques for generating the range image have been known.

For example, as a passive range imaging sensor, a stereo imaging technique for obtaining a distance to an object based on parallax of imaging devices, has been known. Also, as an active range imaging sensor, a light sectioning technique for obtaining a distance to an object based on a triangulation principle, a time-of-flight technique for measuring a time until light reflected from an object is received after being projected, and the like have been widely employed. A range imaging sensor for obtaining a three-dimensional shape of a surface of an object by projecting light patterns having different spatial phases and then using location relationships between the light patterns formed on the surface of the object has also been known.

Although the technique for generating a range image is not specifically limited in the following embodiments, the embodiments will be described using an exemplary case where a range imaging sensor using a time-of-flight technique is used. Hereinafter, the term "time-of-flight thechnique" is abbreviated to "TOF." Various range imaging sensors using TOF have been known. Here, after intensity-modulated light, in which the intensity of light is modulated into a modulated signal having a constant period, is projected to a target space, a time period until the intensity-modulated light reflected from an object existing in the target space is received is detected as a phase difference between the projected intensity-modulated light and the reflected intensity-modulated light, and then, the phase difference is converted into a distance to the object.

The light projected to the target space is not transmitted through a large number of objects but reflected from surfaces of the objects. In the meantime, the light that is not perceived by a person is preferable. On this account, it is preferred that near-infrared light is used as the light to be projected. However, it is possible to use visible light if it is preferred that the light is perceived by a person, such as in a case where an imaging region is controlled.

Although a waveform of the intensity-modulated light is supposed to be a sinusoidal wave, a triangular wave, sawtooth wave, square wave or the like may be used. When a sinusoidal wave, triangular wave, or sawtooth wave is used, the intensity-modulated light is to have a constant period. In addition, when a square wave is used, a technique of randomly varying a ratio of ON durations (light-projecting durations of a light emitting source) to OFF durations (non-light-projecting durations of the light emitting source) can be employed, in addition to the use of the intensity-modulated light having a constant period. That is, for a sufficiently long time for the ON durations and the OFF durations, the ON durations and the OFF durations may be irregularly varied so that a probability that the ON durations occur is 50%, and the quantity of received light accumulated for the sufficiently long time may be used.

Also, when the intensity-modulated light is to have a constant period, for example, the projected light is modulated by a modulation signal of 20 MHz and the quantity of received light is accumulated for about 10000 periods, thereby reducing an influence of shot noise. Even when the ON durations and the OFF durations are randomly varied, for example, with a unit duration being a duration ($5 \times 10^{-8}$s) equivalent to one period of 20 MHz, the ON durations and the OFF durations are varied in a range of several times of the unit duration, the quantity of received light is accumulated for duration of about 10000 times of the unit duration. By this operation, the accumulated quantity of received light may be treated similarly to a case where the quantity of received light is accumulated using intensity-modulated light of a constant period.

The intensity-modulated light reflected from the object is received by an imaging device with pixels arranged two-dimensionally. The imaging device includes an imaging element for taking a gray scale image, and a light reception optical system for limiting a range in which light is incident on a light reception surface of the imaging element. The imaging element includes an imaging element having a well-known configuration for taking a gray scale image, which is provided as a CCD image sensor or a CMOS image sensor. However, it is preferred to use a dedicated imaging element designed to have a suitable structure for the range imaging sensor.

Hereinafter, although the following configuration will be described as an example of the range imaging sensor to better understanding, the configuration does not intend to limit the present invention. For a modulation waveform of intensity-modulated light, the configuration of the imaging element, the control of the imaging element, and the like, the configuration provided in various well-known range imaging sensors may be used.

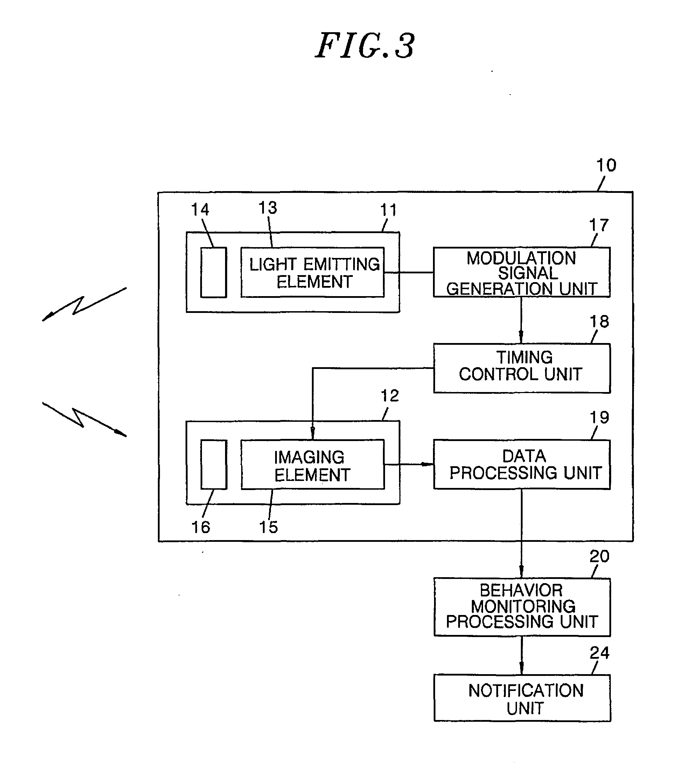

The range imaging sensor 10 used in the following description includes a light projection device 11 for projecting light to a target space, and an imaging device 12 for receiving light from the target space, as shown in Fig. 3. The light projection device 11 is provided with light emitting elements 13 capable of having an optical output in proportion to an instantaneous input value, such as a light emitting diode or laser diode, and a light projection optical system 14 for projecting the light emitted from the light emitting elements 13 into a range of the target space. The light projection device 11 includes a suitable number of the light emitting elements 13 in order to secure an optical output. Also, the imaging device 12 includes an imaging element 15 as described above, and a light reception optical system 16 defining a field of vision of the imaging element 15.

The intensity-modulated light emitted from the light emitting elements 13 passes through the light projection optical system 14 and then is projected into the target space. The imaging element 15 receives the light passing through the light reception optical system 16 from the target space. The light projection optical system 14 and the light reception optical system 16 are disposed adjacent to each other such that the projected light and the received light are parallel with each other. Here, a distance between the light projection optical system 14 and the light reception optical system 16 is substantially negligible relative to the field of vision.

The range imaging sensor 10 includes a modulation signal generation unit 17 for generating a modulation signal to be transmitted to the light emitting elements 13 to make the light emitting elements 13 output the intensity-modulated light. The range imaging sensor 10 also includes a timing control unit 18 for generating a light reception timing signal, which defines a light reception timing of the imaging element 15, from the modulation signal, to control the timing at which the imaging element 15 receives the light from the target space. Electrical charges corresponding to the quantity of the light received by the imaging element 15 are transmitted from the imaging element 15 into a data processing unit 19. The data processing unit 19 obtains a distance to the object existing in the target space based on the relationship between the light reception timing and the quantity of the received light.

The modulation signal generation unit 17 generates a modulation signal having an output voltage in a sinusoidal waveform of a constant frequency (for example, 20 MHz). The light emitting elements 13 are driven by the modulation signal, so that the intensity-modulated light having the optical output in a sinusoidal waveform is emitted from the light emitting elements 13.

The imaging element 15 employed in this embodiment uses an electronic shutter technique, so that electrical charges are generated according to intensity of the received light only during the duration synchronized with the light reception timing signal. In addition, the generated electrical charges are transmitted into a light-shielded accumulation region, accumulated in the accumulation region for accumulation durations corresponding to multiple periods (for example, 10000 periods) of the modulation signal, and then taken out from the imaging element 15 as a light reception output.

The timing control unit 18 generates a light reception timing signal synchronized with the modulation signal. Here, the timing control unit 18 generates four kinds of light reception timing signals having light reception durations of a constant time width for four different phases of the modulation signal. Also, one kind of the light reception timing signal selected from the four kinds of the light reception timing signals for the respective aforementioned accumulation durations is provided to the imaging element 15.

That is, one kind of the light reception timing signal for each of the accumulation durations is provided to the imaging element 15, so that electrical charges are generated in each pixel of the imaging element 15 for the light reception duration corresponding to a specific phase duration of the modulation signal. The accumulated electrical charges are taken out from the imaging element 15 as a light reception output. If the different light reception timing signals are respectively provided to the imaging element 15 every accumulation duration and the operation of taking out the electrical charges generated from the imaging element 15 as the light reception output is repeated, the light reception outputs corresponding to four kinds of the light reception timing signals can be obtained from the imaging element 15 for four accumulation durations.

Now, suppose the four kinds of the light reception timing signals are set up at phases different by 90 degrees for one period of the modulation signal and the light reception outputs (the quantities of electrical charges) output from the imaging element 15 corresponding to the respective light reception timing signals are respectively referred to as A0, A1, A2, and A3. In this case, using trigonometric function relationships, a phase difference ψ [rad] when projecting and receiving the intensity-modulated light may be represented by the following formula:

$$\Psi = (A0 - A2)/(A1 - A3).$$

Since the frequency of the modulation signal is constant, the phase difference ψ may be converted into a time difference from the light projection to the light reception, and since the light speed is a given value, a distance to an object can be obtained if the time difference is obtained.

That is, the distance to the object can be obtained by the four kinds of the light reception outputs (the quantities of electrical charges) A0 to A3. Also, the light reception duration may be set up to be suitable so that a suitable quantity of received light can be obtained in each pixel (for example, it is possible to use a light reception duration corresponding to a quarter of a period of the modulation signal). It is necessary to equalize the time widths of the respective light reception durations to each other.

In the data processing unit 19, in addition to the aforementioned processing of obtaining the phase difference ψ based on the light reception outputs (the quantities of electrical charges) A0 to A3 and converting the phase difference ψ into the distance, the processing that will be described in the following embodiments is also performed. The data processing unit 19 is configured to include a digital signal processing device selected from a micro computer, DSP, FPGA and the like, and the aforementioned processing is implemented by executing a program in the digital signal processing device. In addition, the data processing unit 19 and the configurations except for the light emitting elements 13 and the imaging element 15 can be implemented using the aforementioned digital signal processing device. Although it is possible to use only one digital signal processing device, it is preferred that at least the function used in the range imaging sensor 10 and the functions that will be described later are processed by another digital signal processing device. Also, it is possible to use three or more digital signal processing devices.

Further, although the four kinds of the light reception timing signals are used in the aforementioned exemplary operation, a phase difference ψ can be obtained from three kinds of light reception timing signals. Under an environment in which environment light or ambient light does not exists, it is possible to obtain a phase difference ψ from two kinds of light reception timing signals.

Furthermore, in the aforementioned operation, since electrical charges corresponding to one kind of light reception timing signal are accumulated for one pixel, four accumulation durations are needed to take out four kinds of the light reception outputs (the quantities of electrical charges) A0 to A3 from the imaging element 15. Meanwhile, if electrical charges corresponding to two kinds of light reception timing signals are accumulated for one pixel, it is possible to read out the light reception outputs corresponding to two kinds of the light reception timing signals from the imaging element 15 at a time. In the same manner, if electrical charges corresponding to four kinds of the light reception timing signals are accumulated for one pixel, it is possible to read out the light reception outputs corresponding to four kinds of the light reception timing signals at a time.

Since the aforementioned range imaging sensor 10 uses an imaging element having pixels two-dimensionally arranged as a light reception element for receiving light from the target space, the range image can be generated by obtaining distance values as pixel values of the respective pixels. That is, the light reception surface of the imaging element becomes an imaginary projection surface on which the field of vision of the range imaging sensor 10 is projected.

Since the aforementioned range imaging sensor 10 takes an image of the field of vision of the imaging element 15 as the target space by projecting the light from the light emitting elements 13 into the target space, the target space has a shape which is widened as it goes away from the range imaging sensor 10 with the range imaging sensor 10 as an apex. For example, if the light projection optical system 14 and the light reception optical system 16 are isotropically formed around the respective optical axes, the target space has a pyramid shape with the range imaging sensor 10 as an apex.

Therefore, locations of the pixels arranged on the aforementioned imaginary projection surface correspond to directions viewing the target space from the range imaging sensor 10, and the pixel value of each pixel represents a distance to an object existing in the corresponding direction. In other words, the range image generated from the range imaging sensor 10 (and an image generating unit) represents the location of the object in a polar coordinate system. Such a range image is referred to as a range image of a polar coordinate system.

Although the aforementioned range image of a polar coordinate system is convenient when information of a distance from the range imaging sensor 10 is required, it is difficult to know a corresponding relationship with the location of the real space that is the target space, and it is inconvenient to appoint a region with respect to an object existing in the real space. Therefore, the coordinate transformation for generating an image having respective coordinate values of a Cartesian coordinate system from a range image of a polar coordinate system is performed in the data processing unit 19. Hereinafter, the image subjected to the coordinate transformation will be referred to as a coordinate axis-based image.

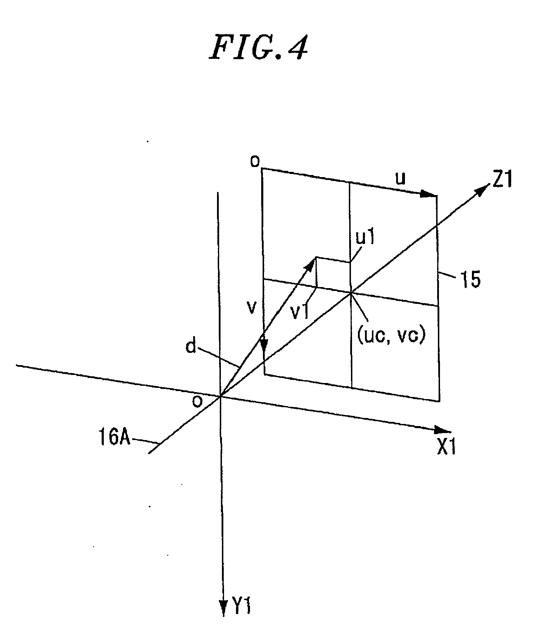

A procedure of generating a coordinate axis-based image from a range image of a polar coordinate system will be described with reference to Figs. 4 to 5C. In order to generate a coordinate axis-based image from a range image of a polar coordinate system, a range image of a polar coordinate system is first transformed into a three dimensional image of a camera coordinate system that is a Cartesian coordinate system, and this three dimensional image is then transformed into a three dimensional image of a global coordinate system that is a Cartesian coordinate system defined in the target space. If the three dimensional image of a global coordinate system can be obtained, it can be decomposed into respective components of the coordinate axis-based image.

In order to generate a three dimensional image of a camera coordinate system from a range image of a polar coordinate system, the following operation is performed. Here, as shown in Fig. 4, suppose a horizontal direction and a vertical direction of the light reception surface of the imaging element 15 are a u direction and a v direction, respectively. The imaging element 15 and the light reception optical system 16 are disposed so that an optical axis 16A of the light reception optical system 16 passes through a pixel at the central location of the light reception surface of the imaging element 15. Also, the light reception surface of the imaging element 15 is located at a focus of the light reception optical system 16. In this positional relationship, suppose coordinates (unit: pixel) of the pixel at the central location of the light reception surface of the imaging element 15 are (uc, vc), and pitches (unit: mm) in u and v directions of pixels of the imaging element 15 are (su, sv). Also, suppose a focal length of the light reception optical system 16 is f [mm], and a distance from the center of the light reception optical system 16 to an object, which exists in a direction corresponding to the location (u, v) of coordinates (unit: pixel) of each pixel on the light reception surface of the imaging element 15, is d [mm]. These values become given values by allowing the distance d to the object from the range imaging sensor 10 to correspond to the location of each pixel.

Coordinate values (X1, Y1, Z1) of the object in a camera coordinate system may be obtained as follows. A unit of each component of the coordinate values (X1, Y1, Z1) is mm. Also, suppose the starting point of the coordinate system set up in the light reception surface of the imaging element 15 is a location of one corner of the rectangular light reception surface of the imaging element 15, and the starting point of the Cartesian coordinate system is the center of the light reception optical system 16.

$$X1 = u1 \cdot d/R$$

$$Y1 = v1 \cdot d/R$$

$$Z1 = f \cdot d/R$$

wherein

$$u1 = su(u - uc)$$

$$v1 = sv(v - vc)$$

$$R = (u1^2 + v1^2 + f^2)^{1/2}$$

Here, for ease of explanation, an influence of optical distortion of the light reception optical system 16 is ignored. However, the optical distortion can be corrected using a distortion correction formula for correcting a distance R from an optical center.

As described above, after the range image of a polar coordinate system is transformed into the three dimensional image of a camera coordinate system, the coordinate transformation into a global coordinate system is performed. The range imaging sensor 10 is disposed so that the vertical direction (that is, v direction) of the light reception surface of the imaging element 15 is in parallel with a wall surface 51 and a floor surface 52 in a room.

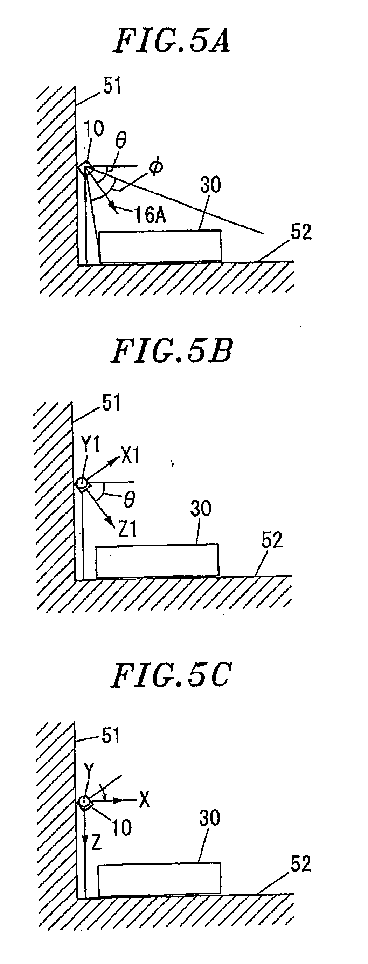

Now, as shown in Fig. 5A, suppose a depression angle of the optical axis 16A of the light reception optical system 16 in the range imaging sensor 10 is θ. In Fig. 5A, a viewing angle of the range imaging sensor 10 is designated by φ. In the transformation from a camera coordinate system into a global coordinate system, as shown in Fig. 5B, the coordinate values (X1, Y1, Z1) in the camera coordinate system are rotated by the depression angle θ about the Y-axis. Accordingly, as shown in Fig. 5C, a three dimensional image of a global coordinate system having coordinate axes perpendicular to the wall surface 51 and the floor surface 52 in the room is generated.

Hereinafter, suppose coordinate values in the global coordinate system are (X, Y, Z). In the shown example, a direction perpendicular to and away from the wall 51 is a positive direction of the X axis, and a downward direction perpendicular to the floor surface 52 is a positive direction of the Z axis. A right handed coordinate system is also used. The rotation of the coordinate system by the depression angle θ is performed by the following calculations:

$$X = X1 \cdot \cos(90° - \theta) + Z1 \cdot \sin(90° - \theta)$$

$$Y = Y1$$

$$Z = -X1 \cdot \sin(90° - \theta) + Z1 \cdot \cos(90° - \theta)$$

The coordinate axis-based image is not an image obtained by performing the mapping to the global coordinate system but an image having the X, Y, and Z values individually corresponding to a coordinate location (u, v) of each pixel of the range image. That is, the coordinate axis-based image is an image having X (u, v), Y (u, v) and Z (u, v) respectively corresponding to each coordinate location (u, v), and the coordinate axis-based image having three coordinate values is obtained for a single range image of a polar coordinate system. In order to obtain the coordinate axis-based image from the range image of a polar coordinate system, the aforementioned calculation may be performed. However, if a table of transforming a range image of a polar coordinate system into coordinate values (X, Y, Z) is prepared, it is possible to reduce a processing load.

Hereinafter, an image in which a pixel value is an X value of a coordinate axis-based image is referred to as an X image, an image in which a pixel value is a Y value thereof is referred to as a Y image, and an image in which a pixel value is a Z value thereof is referred to as a Z image. Therefore, a coordinate transformation table includes three kinds of tables, i.e., an X transformation table for transformation into an X image, a Y transformation table for transformation into a Y image, and a Z transformation table for transformation into a Z image. A technique of using a coordinate axis-based image will be described later.

The aforementioned X image, Y image and Z image are stored in a memory of the digital signal processing device, and a three-dimensional imaginary space corresponding to the target space that is a real space is represented. Therefore, if conditions in the imaginary space are set up, this becomes the same as setting up conditions in the target space.

The X image, Y image and Z image generated from the range imaging sensor 10 are applied to a behavior monitoring processing unit 20. The behavior monitoring processing unit 20 classifies movement of an object person 40 to be monitored in relation to the bed 30. In this embodiment, a case where the bed 30 disposed in a hospital room or a room of a nursing facility for the elderly or care facility is monitored is supposed. The movement to be classified will be described later.

In order to classify the movement of the object person 40 as described above, it is necessary to extract the bed 30 regardless of whether or not the object person 40 exists on the bed 30. Also, in order to monitor the object person 40 on the bed 30, the range imaging sensor 10 is disposed at a location from which the bed 30 is viewed down. In order to satisfy this condition, the range imaging sensor 10 may be disposed at a ceiling, but there is a problem in that a construction work, in which the range imaging sensor 10 is installed to the ceiling of the interior, requires a great deal of care.

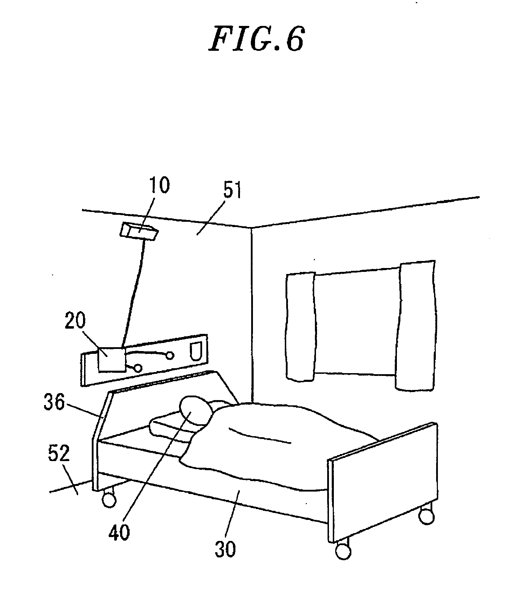

Therefore, as shown in Fig. 2B, a configuration in which the range imaging sensor 10 is attached to a top end of a stand 31 standing on a floor may be employed. Alternatively, as shown in Fig. 6, a configuration in which the range imaging sensor 10 is attached to one end of an arm (not shown) having the other end attached to the wall 51 may be employed. In the configuration shown in Fig. 2, the stand 31 has a post plate 35 attached on a base plate 34 having casters 33, and the range imaging sensor 10 is attached to a top end of the post plate 35. Also, the behavior monitoring processing unit 20 is provided in an intermediate portion of the post plate 35.

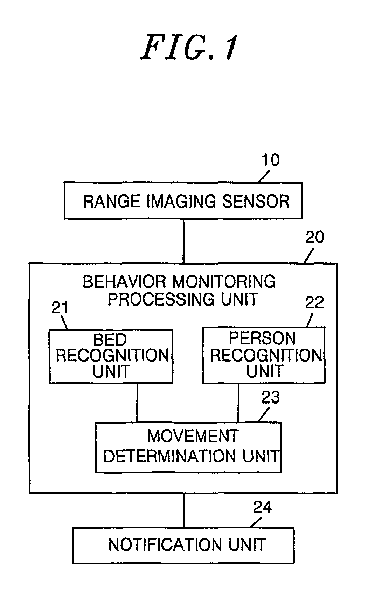

The behavior monitoring processing unit 20 notifies monitored information to an inside or outside of a room through a notification unit 24 (see Figs. 1 and 3). When the monitored information is notified to the outside, communications are carried out using a local area network (LAN) or nurse call system network. For example, when the stand 31 is installed in a hospital room, a reception device for receiving the notification of the notification unit 24 is provided in a nurse center, so that it is possible to notify the reception device provided in the nurse center of the movement of the object person classified in the behavior monitoring processing unit 20. Hereinafter, the notification to the reception device provided in the nurse center means that "the nurse center is notified" or "the nurse center is informed."

Since the range imaging sensor 10 and the behavior monitoring processing unit 20 are provided on the stand 31 having the casters 33 so that they are movable, it is possible to move them to a required location. That is, the stand 31 need not be carried into a room in which the movement of the object person 40 need not be monitored. In addition, even though the movement of the object person 40 is monitored, since the stand 31 may be disposed between a headboard 36 of the bed 30 and the wall surface 51 as shown in Fig. 2A, it is no problem that the stand 31 is disposed.

As is clear from the aforementioned configuration, the range imaging sensor 10 is disposed higher than the bed 30. In practice, the range imaging sensor 10 is disposed at a height so that the entirety of the bed 30 is included in the field of vision that is the target space. The disposition of the range imaging sensor 10 at such a height makes it possible to prevent a person from being carelessly brought into contact with the range imaging sensor 10 and to dispose the range imaging sensor 10 to avoid disturbing a person. For example, when an object person (patient) is treated in a hospital, the stand 31 does not disturb the work. Also, since the range imaging sensor 10 detects a distance to an object existing in the target space in a contactless manner, the movement of the object person is not disturbed and person's sleep on the bed 30 is also not disturbed.

In order to look down at the bed 30 as shown in Fig. 2A, the range imaging sensor 10 is disposed so that its optical axis 16A (see Fig. 5) is obliquely downward. Also, in such a state, an inclination angle (depression angle $\theta$) of the optical axis 16A is set up so that an entire upper surface of the bed 30 is included in the field of vision of the range imaging sensor 10. Here, since the optical axis 16A is obliquely downward, a distance to the upper surface of the bed 30 at a headboard side of the bed 30 is relatively small, while a distance to the upper surface of the bed 30 at a footboard side of the bed 30 is relatively large.

Hereinafter, the behavior monitoring processing unit 20 will be described in detail. As described above, in order to classify movement of the object person 40, it is necessary to extract the bed 30 from the range image output by the range imaging sensor 10. Therefore, the behavior monitoring processing unit 20 includes a bed recognition unit 21 for recognizing the bed 30 using information included in the range image. In addition, since the bed recognition unit 21 recognizes the bed 30, it is possible to recognize whether or not a person exists in the target space using a relative relationship between the information included in the range image and the bed 30.

The behavior monitoring processing unit 20 is provided with a person recognition unit 22 for recognizing whether or not a person exists based on a relationship between the bed 30 recognized by the bed recognition unit 21 and another object. Also, the behavior monitoring processing unit 20 includes a movement determination unit 23, which synthesizes the recognition by the bed recognition unit 21 and the recognition by the person recognition unit 22 to classify movement of the object person and to notify the movement as necessary.

The bed recognition unit 21 first detects an outer periphery of the bed 30 when the bed 30 is viewed in a plan view in order to recognize a location of the bed 30. Since the plan view of the bed 30 is generally in the shape of a rectangle, the bed recognition unit 21 recognizes the location of the bed 30 by extracting four sides of the bed 30.

Here, under the assumption that the lengthwise direction of the bed 30 is perpendicular to the wall 51 as shown in Fig. 5C, the X and Y images are used to extract the four sides of the bed 30. However, such a relationship is only an example. It is not essential that a positional relationship between a direction in which the bed 30 is disposed and the range imaging sensor 10 is the relationship shown in Fig. 5C, but it is possible to change a coordinate transformation method depending on a positional relationship between the range imaging sensor 10 and the bed 30. Also, it is possible to change conditions described below as necessary.

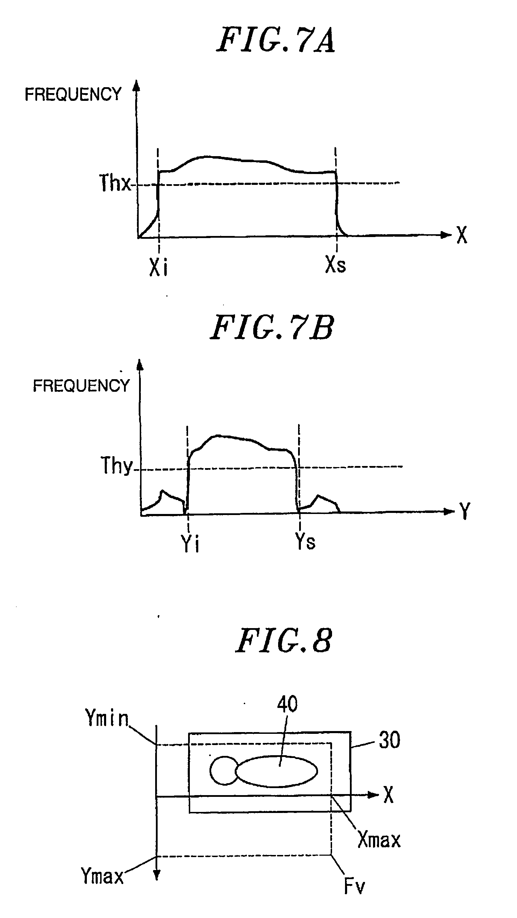

In order to extract the four sides of the bed 30, the X image is used to obtain a frequency distribution of X values, and the Y image is used to obtain a frequency distribution of Y values. Also, in order not to extract an object region other than the bed 30 as far as possible, a suitable threshold value (for example, a height of 15 cm or more from the floor 52) is defined for Z values. In such a case, the frequency distribution of X values is represented, for example as in Fig. 7A, and the frequency distribution of Y values is represented, for example as in Fig. 7B.

That is, if the upper surface of the bed 30 is planar, the same X values are arranged in the widthwise direction of the bed 30, and the same Y values are arranged in the lengthwise direction of the bed 30. In the meantime, since frequencies are rapidly reduced at ends of the bed 30, if frequencies are compared with suitable threshold values Thx and Thy, it is considered that the X and Y values when the frequencies cross the threshold values Thx and Thy become candidates of the respective ends.

Here, since an area of a region of a cabinet, a chair, a medical equipment, or the like having a height from the floor 52 except for the bed 30 is smaller than that of the bed 30, the frequency thereof is smaller than that of the bed 30 (see a portion less than Yi or more than Ys in Fig. 7B). Therefore, the suitable threshold values Thx and Thy for frequency are set up, so that the articles, except for the bed 30, such as a cabinet, a chair, a medical equipment, and the like can be removed.

In addition, whether the ends of the bed 30 are correctly extracted is determined as follows. For the X value, a range greater than or equal to the threshold value Thx is compared with an allowable range set up for a dimension of the bed 30 in the lengthwise direction thereof. Then, if the range greater than or equal to the threshold value Thx is within the allowable range, the maximum value Xs and the minimum value Xi in the range greater than or equal to the threshold value Thx represent locations of the ends of the bed 30 in its lengthwise direction.

For the Y value, a range greater than or equal to the threshold value Thy is compared with an allowable range set up for a dimension of the bed 30 in the widthwise direction thereof. Then, if the range greater than or equal to the threshold value Thy is within the allowable range, the maximum value Ys and the minimum value Yi in the range greater than or equal to the threshold value Thy represent locations of the ends of the bed 30 in its widthwise direction.

The allowable ranges are set up so that suitable ranges are applied to the known dimensions of the bed 30, respectively. For example, for the bed having a dimension in the lengthwise direction of L1 and a dimension in the widthwise direction of W1, the allowable range in the lengthwise direction may be (L1 $\pm$ $\Delta$L) and the allowable range in the widthwise direction may be (W1 $\pm$ $\Delta$W). The values $\Delta$L and $\Delta$W are set up to be suitable values, for example 10 cm, in consideration of a measurement error of the range imaging sensor 10 or the number of pixels.

Also, as described later, when detecting the movement of the object person 40 who comes from and goes to the bed 30, the bed 30 is treated as a rectangle using a parallel projection image to the Z direction. Hence, after the ends of the bed 30 are obtained, assuming that each side of the bed 30 is a straight line, a region inside of a rectangle circumscribed by the ends obtained as described above is a region inside of a range of the bed 30. That is, a rectangular region surrounded by the ends in the lengthwise direction and the ends in the widthwise direction is inside of the range of the bed 30.

The procedure of obtaining the ends of the bed 30 is summarized as follows. First, coordinate locations (u, v), at which for a Z image, Z values are greater than or equal to a suitable threshold value with respect to the floor surface, are obtained. Then, a frequency distribution for X values of the obtained coordinate locations (u, v) is obtained using an X image, and a frequency distribution for Y values of the obtained coordinate locations (u, v) is obtained from a Y image. Then, the frequency distribution of the X values is compared with the threshold value Thx, and the frequency distribution of the Y values is compared with the threshold value Thy.

Thereafter, a range (Xs - Xi), in which a frequency of an X value is greater than or equal to the threshold value Thx, is compared with an allowable range (L1 $\pm$ $\Delta$l) in the lengthwise direction of the bed 30, and a range (Ys - Yi), in which a frequency of a Y value is greater than or equal to the threshold value Thy, is compared with an allowable range (W1 $\pm$ $\Delta$W) in the widthwise direction of the bed 30. If the range, in which a frequency of an X value is greater than or equal to the threshold value Thx, is included in the allowable range (L1 $\pm$ $\Delta$L), X values (Xs, Xi) crossing the threshold value Thx are X coordinates of the ends of the bed 30 in the lengthwise direction thereof. Also, If the range, in which a frequency of a Y value is greater than or equal to the threshold value Thy, is included in the allowable range (W1 $\pm$ $\Delta$W), Y values (Ys, Yi) crossing the threshold value Thy are Y coordinates of the ends of the bed 30 in the widthwise direction thereof.

Also, in the aforementioned example, the ranges (Xs - Xi) and (Ys - Yi), in which frequencies are greater than or equal to the threshold values Thx and Thy, are obtained and then compared with the allowable ranges (L1 $\pm$ $\Delta$L) and (W1 $\pm$ $\Delta$W) , respectively, but the following procedure may be employed.

First, for the frequency distribution of X values, a frequency is compared with the threshold value Thx while an X value is varied in a direction in which it increases from the minimum value. If an X value, in which a frequency is greater than or equal to the threshold value Thx, is continuous for a predetermined value or more, the X value represents one end point of the bed 30 in the lengthwise direction thereof. In addition, a frequency is compared with the threshold value Thx while an X value is varied in a direction in which it decreases from the maximum value. If an X value, in which a frequency is greater than or equal to the threshold value Thx, is continuous for a predetermined value or more, the X value represents the other end point of the bed 30 in the lengthwise direction thereof. Although the continuity is preferably determined based on the allowable range (L1 $\pm$ $\Delta$L), a smaller value may be used. In this way, both the ends of the bed 30 in the lengthwise direction thereof can be determined. The locations of both the ends of the bed 30 in the widthwise direction thereof are also determined in the same manner, wherein Y values may be used instead of the X values and the threshold value Thy may be used instead of the threshold value Thx.

As described above, the other article near the bed 30 is smaller than the bed 30 in terms of a region occupied by the extracted pixels having Z values less than or equal to the predefined threshold value (away from the floor 52). Since the ends of the bed 30 are obtained using frequencies of the X and Y values of the pixels based on such a condition, it is possible to accurately obtain the ends of the bed 30 without affecting the other articles near the bed 30. In addition, when the pixel satisfying the condition in the aforementioned processing does not exist, there is a possibility that the bed 30 is misaligned in the field of vision of the range imaging sensor 10. Therefore, the notification unit 24 preferably notifies that the range imaging sensor 10 is unsuitably positioned.

However, although each end of the bed 30 is included in the field of vision of the range imaging sensor 10, the movement of the object person 40 cannot be determined if a periphery of the field of vision has a margin. With that, in order to secure that a region other than the bed 30 is included around the bed 30 in the field of vision of the range imaging sensor 10, the following determination is carried out.

$$\text{Xmax - Xs} \geq \text{Mgx1}$$

$$\text{Xi - Xmin} \geq \text{Mgx2}$$

$$\text{Ymax - Ys} \geq \text{Mgy1}$$

$$\text{Yi - Ymin} \geq \text{Mgy2}$$

Here, Xmax is a maximum value of the X values in the X direction of the field of vision, Xmin is a minimum value of the X values.in the X direction of the field of vision, Ymax is a maximum value of the Y values in the Y direction of the field of vision, and Ymin is a minimum value of the Y values in the Y direction of the field of vision. In addition, allowable dimensions set up around the bed 30 are Mgx1, Mgx2, Mgy1, and Mgy2. In such a case, since the allowable dimensions Mgx1, Mgx2, Mgy1, and Mgy2 become different values due to the disposition of the range imaging sensor 10 with respect to the bed 30 or the disposition of the bed 30 indoors, they can be preferably set up by a setup means not shown. In addition, in this embodiment, since the stand 31 provided with the range imaging sensor 10 is disposed between the headboard of the bed 30 and the wall surface (see Fig. 1), it may be unnecessary to use the allowable dimension Mg2 of the headboard side.

If any one of the conditions of the aforementioned determination is not satisfied, it is determined that the field of vision Fv is misaligned with the bed 30 as shown in Fig. 8, and the notification unit 24 preferably notifies that the range imaging sensor 10 is unsuitably positioned. In the example shown in Fig. 8, Xmax is equal to Xs since one end of the bed 30 in the lengthwise direction thereof is not included in the field of vision Fv, and Ymin is equal to Yi since one end of the bed 30 in the widthwise direction thereof is not included in the field of vision Fv. Therefore, the conditions, Xmax - Xs ≥ Mgx1 and Yi - Ymin ≥ Mgy2, are not satisfied. Such determination is performed, so that it is possible to monitor the movement of the object person 40 who comes from and goes to the bed 30.

As described above, if the location of the bed 30 is recognized by the bed recognition unit 21, the location of the upper surface of the bed 30 is then detected. Hereinafter, the upper surface of the bed 30 is referred to as a bed surface. In connection with the detection of the height of the bed surface, suppose a state where the object person 40 sleeps on the bed 30 (which is referred to as a "bed-ridden state"). In a state where the object person 40 lies on the bed, the object person 40 and bedding such as a blanket are placed on the bed 30. Although the downward direction in the real space is a positive direction of the Z axis in the aforementioned global coordinate system, when the height in the real space is described, the upward direction is taken as a direction in which the value increases. That is, the Z direction in which a Z value increases is treated as a direction in which a height decreases.

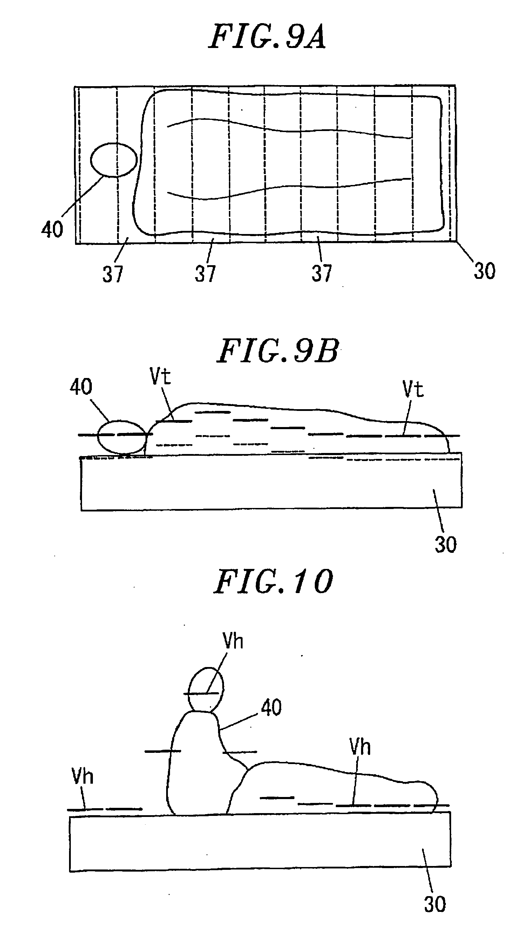

In the bed recognition unit 21, the range of the bed 30 is segmented into a predefined number (for example, ten) of segmented regions 37 in the lengthwise direction, as shown in Fig. 9A. The number of the segmented regions 37 may depend on a dimension of the bed 30 in the lengthwise direction thereof. In addition thereto, the number is determined so that one of the segmented regions 37 has a width of about 10 to 20cm. Then, a representative value of height of each segmented region 37 is obtained. As the representative value, any one of an average value and a mode value may be used.

When using an average value, locations (u, v) belonging to each segmented region 37 are obtained from the Z image, and an average value of Z values is obtained for each segmented region 37. In the meantime, when using a mode value, locations (u, v) belonging to each segmented region 37 are obtained for the Z image, an frequency distribution of Z values is obtained for each segmented region 37, and a mode value is then obtained from the obtained frequency distribution. It is considered that in this frequency distribution, a frequency of the bed surface or a frequency of an upper surface of the bedding increases and then a frequency corresponding to the object person 40 increases although it depends on the location of the segmented regions 37. This is based on the knowledge that on the bed 30, a region (area) occupied by the bed surface or the bedding is larger than that occupied by the object person 40.

If the representative values of height are obtained from the respective segmented regions 37 as described above, the representative values Vt are distributed as shown by solid lines in Fig. 9B. In addition, a value obtained by subtracting an offset value considering a person's thickness from (or adding a Z value to) the representative values Vt is used as the height of the bed surface. Although the person's thickness has a difference between individuals, for example, 20 cm or the like is used as the person's thickness and a half thereof is used as the offset value. Since there is no special condition for the offset value, the offset value is set up to be suitable so that the values obtained by subtracting the offset value from the representative values represent the heights of the bed surface in the segmented regions 37. Broken lines in Fig. 9B represent, for example, the values obtained by subtracting the offset value from the representative values Vt. In this way, the heights of the bed surface obtained for the respective segmented regions 37 become reference heights for the respective segmented regions 37.

In the subsequent processing, a set of bed surfaces for the respective segmented regions 37 is treated as the bed surface of the entirety of the bed 30. In addition, since not the entirety of the bed 30 but the segmented regions 37 segmented in the lengthwise direction are used, the bed surfaces obtained for the respective portions of the bed 30 are used to make it possible to separate the object person 40 therefrom, even when the bed 30 is a reclining type and the bed surface is not planar.

However, although the height of the bed surface is obtained on the supposition that the object person 40 on the bed 30 is in a bed-ridden state in the aforementioned example, when the object person 40 sits on the bed 30 as shown in Fig. 10, it is hard to say that the height of the bed surface obtained as described above (which is represented by Vh in Fig. 10) is suitable. Therefore, an upper limit of height is provided for each segmented region 37, and when a value obtained as a height Vh of the bed surface exceeds the upper limit, the notification unit 24 notifies that the obtained value cannot be employed as the height Vh of the bed surface.

Here, since the measurement of the bed surface of the bed 30 is performed immediately after the start of the system's operation is instructed, it is possible to perform a suitable action by the notification of the notification unit 24. In addition, the instruction of the start of the system's operation means operation of turning on a switch (not shown) for starting the operation. Also, the suitable action means that the instruction of the start of the system's operation is provided again after the object person 40 is into a bed-ridden state in a proper place.

Although the aforementioned bed 30 is considered to have the bed surface having a fixed height, in order to make easy nursing or care, an elevation type bed 30 capable of changing the height of the bed surface is also provided. Since the bed surface of the elevation type bed 30 is higher than usual if it is forgotten to lower the bed surface after the nursing or care, it is difficult for the object person 40 to land feet on the floor when he or she leaves the bed 30, and the object person 40 is immensely shocked when he or she falls from the bed 30. Therefore, it is necessary to always lower the bed surface after the nursing or care.

When the bed 30 is an elevation type, since it is thought that a lower limit of the bed surface exceeds a predetermined threshold height value Thh during the nursing or care, it is possible to detect the elevation of the bed surface by setting up the suitable threshold height value Thh. That is, when the heights of the bed surfaces obtained for all the respective segmented regions 37 are larger than the threshold height value Thh, it is determined that the bed surface is raised, which is notified by the notification unit 24.

Meanwhile, during the nursing or care, since it is unnecessary to monitor the object person 40, the operation of the system is stopped. Then, after the nursing or care, the operation of the system is restarted. Therefore, when the restart of the system's operation is instructed after the nursing or care is ended, the abnormality of the height of the bed surface is notified, and thus, it is possible to prevent lowering the bed surface from being forgotten.

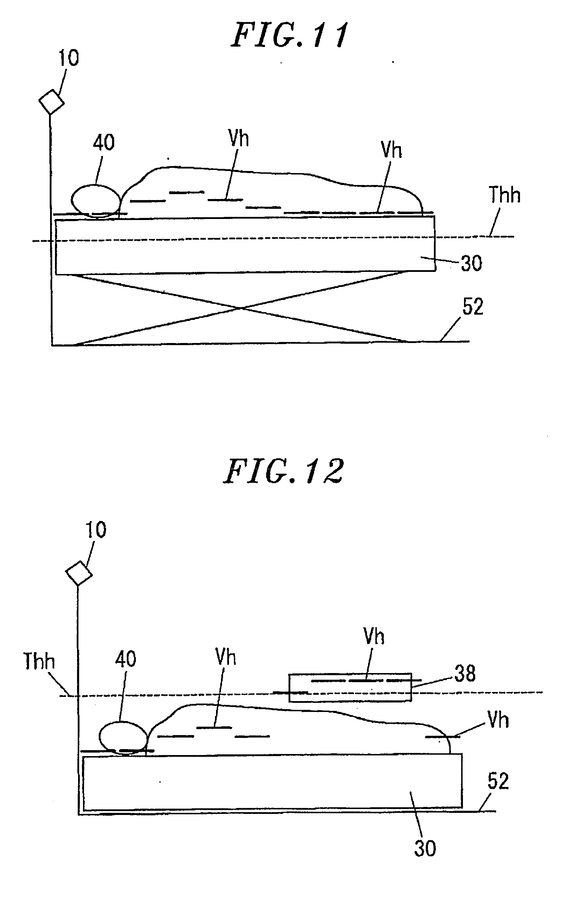

In addition, when a table 38 is placed on the bed 30 as shown in Fig. 12, it is sometimes forgotten to pick up the table 38. In such a case, if a threshold height value Thh for the segmented regions 37 corresponding to the table 38 is set up so that the height of the table 38 may be detected, the notification unit 24 can notify that it is forgotten to pick up the table 38.

In a case where it is detected both that it is forgotten to lower the bed 30 and that it is forgotten to pick up the table 38, if the suitable threshold height value Thh is set up and the height Vh of a predetermined number or more of the segmented regions 37 exceeds the threshold height value Thh, the notification unit 24 may provide the notification therefor. Since the determination using the threshold height value Thh is performed directly after the start of the system's operation is instructed like the measurement of the bed surface, when a problem occurs, the notification unit 24 immediately provides the notification therefor. Therefore, the suitable action can be made, such as lowering the bed 30 or picking up the table 38. Thereafter, the start of the system's operation may be instructed again.

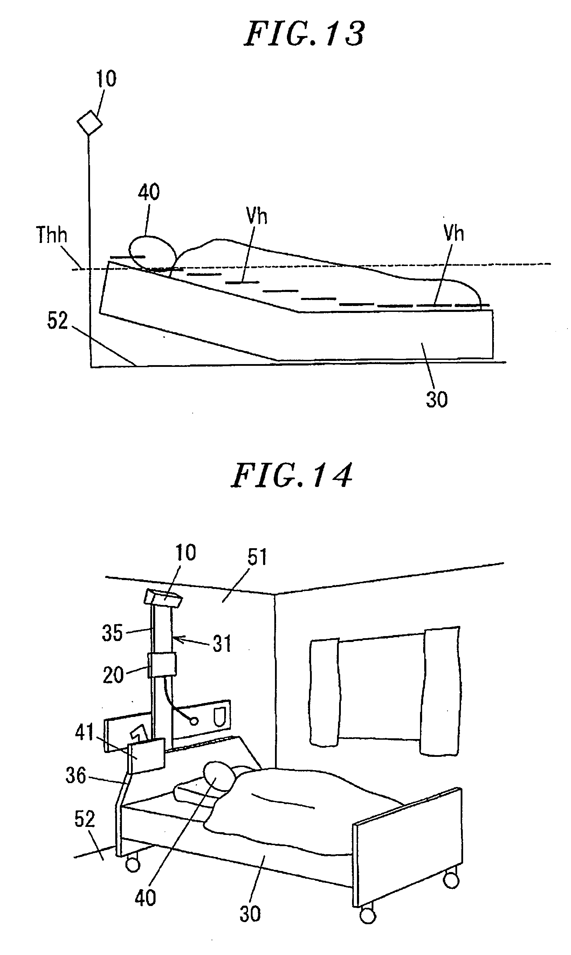

When the bed 30 has a reclining function and the upper body of the object person 40 is bent forward as shown in Fig. 13, the determination by the threshold height value Thh may be performed only for a plural number (five in the illustrated example) of the segmented regions 37 corresponding to the lower body side. Since the location of the bed 30 in the range image is known, for example, the determination by the threshold height value Thh may be performed only the segmented regions 37 having an X value larger than (Xs + Xi)/2. Since a range in which the determination by the threshold height value Thh is performed as described above can be formulated and then automatically determined, no manual adjustment work is necessary.

In the meantime, a variety of devices are often disposed around the bed 30. For example, as shown in Fig. 14, an object 41 other than the object person 40 may be often disposed on an upper side of the bed 30 so that they overlap each other. This kind of the object 41 includes, for example, a lighting stand, a monitoring device, and various medical devices.

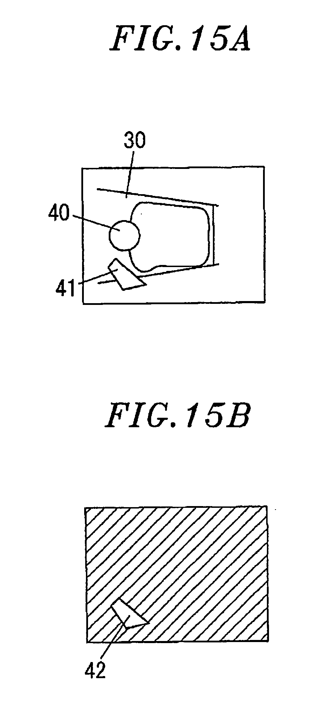

When such an object 41 overlaps the bed 30, it is necessary to exclude the object 41 from the region in which the object person 40 is detected. Hence, when a nurse or caregiver operates the device and thus the bed recognition unit 21 is allowed to recognize the location of the bed 30, the processing of excluding the region of the object 41 from the region of the bed 30 may also be performed. That is, in the field of vision of the range imaging sensor 10, the region in which the object 41 overlaps the bed 30 may be memorized as a mask region 42, which is excluded from the region of the bed 30, as shown in Figs. 15A and 15B. When the person recognition unit 22 recognizes the object person 40, the mask region 42 is treated as a region outside of the range of the bed 30, so that it is possible to prevent the object 41 from being mistaken for the object person 40.

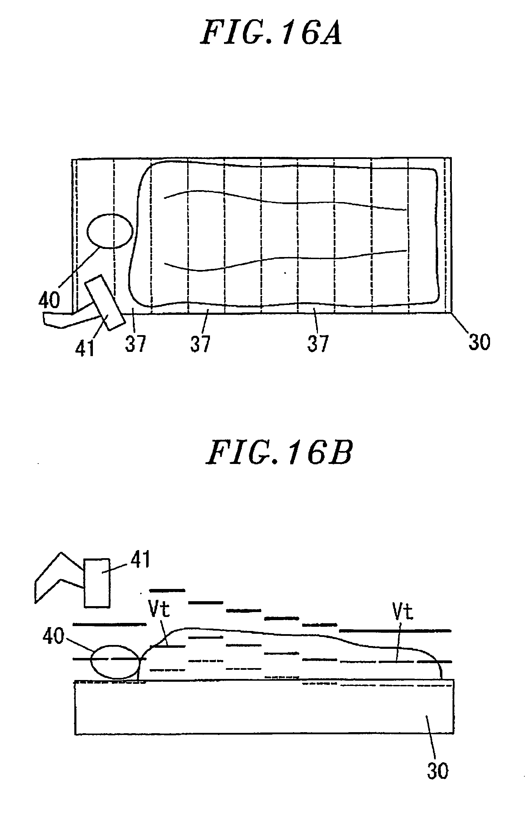

The mask region 42 is set up immediately after the bed recognition unit 21 extracts the location of the bed 30. That is, the nurse or caregiver operates the device, whereby the bed recognition unit 21 determines whether or not the mask region 42 exists immediately after the location of the bed 30 is recognized. As shown in Fig. 16, the object 41 is located in the range of the bed 30 and above a predefined exclusion threshold value for a reference height (height of the bed surface) obtained for each segmented region 37, the bed recognition unit 21 recognizes the range in which the object 41 exists as the mask region 42. The exclusion threshold value is set up to be suitable based on the actual situation. Since the mask region 42 set up in this manner is treated as the region excluded when the object person 40 is recognized, the mask region 42 is memorized in the bed recognition unit 21.

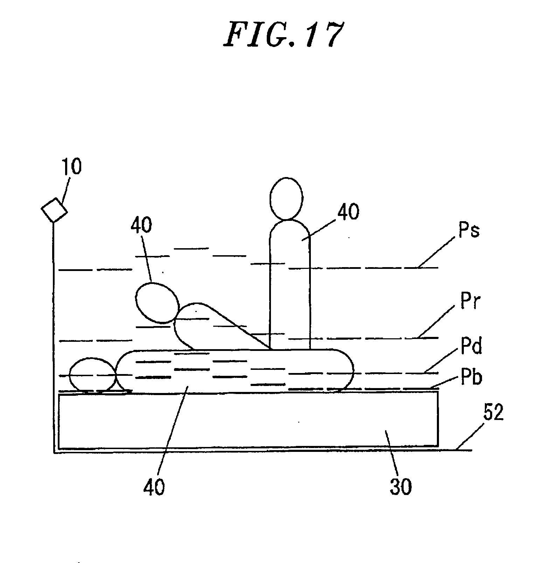

In the aforementioned operation, the height of the bed surface is used as the reference height for each segmented region 37. However, since the object person 40 exists on the bed 30 when the bed recognition unit 21 recognizes the location of the bed 30, it is preferred that a bed-ridden state detection plane Pd (see Fig. 17), which will be described later, is used as the reference height. The bed-ridden state detection plane Pd is a reference height in a state where the object person 40 lies straight on his or her back on the bed 30. In this way, if the bed-ridden state detection plane Pd is used as the reference height, it is possible to prevent the region in which the object person 40 exists from being mistaken for the mask region 42. However, when the bed-ridden state detection plane Pd is defined as a predetermined height from the bed surface, the use of the bed-ridden state detection plane Pd as the reference height is identical to the use of the bed surface as the reference height.

In addition, when a size of the mask region 42 is estimated and the mask region 42 having a size exceeding a predefined threshold value exists, it is determined that a table or the like is placed. In such a case, it is not possible to maintain the reliability when the object person 40 is recognized, and there is a possibility that the object person 40 is in contact with the object 41. Thus, instead of setting up the mask region 42, it is preferred that the notification unit 24 notifies that the object 41 is poorly installed.

Hereinafter, a technique of recognizing the object person 40 by the person recognition unit 22 will be described. As described above, since the bed recognition unit 21 detects the bed surface, the person recognition unit 22 sets up multiple steps of detection planes using the bed surface as a reference. Here, three states, i.e., a bed-ridden state, a sitting state with the upper body bent forward, and a rising stand-up state, are considered as states on the bed. In order to detect each state, a height of each segmented region 37 defined as a bed surface Pb is used as a reference as shown in Fig. 17, and a bed-ridden state detection plane Pd, a sitting detection plane Pr, and a stand-up detection plane Ps are set up.

Each of the bed-ridden state detection plane Pd, the sitting detection plane Pr, and the stand-up detection plane Ps is set up by displacing the bed surface Pb only by a predetermined amount in the Z direction. That is, since the bed surface Pb has the determined Z value for each segmented region 37, each of the bed-ridden state detection plane Pd, the sitting detection plane Pr, and the stand-up detection plane Ps is also determined by being spaced upwardly from the bed surface Pb by a predetermined distance. Also, distances from the bed surface Pb to the bed-ridden state detection plane Pd, the sitting detection plane Pr, and the stand-up detection plane Ps are increased in order. As an example, the bed-ridden state detection plane Pd is set up 10 cm upward from the bed surface Pb, the sitting detection plane Pr is set up 30 cm upward from the bed surface Pb, and the stand-up detection plane Ps is set up 60 cm upward from the bed surface Pb. The bed-ridden state detection plane Pd, the sitting detection plane Pr, and the stand-up detection plane Ps are used as threshold values for detecting the location of the person on the bed 30.

The person recognition unit 22 determines that the object person 40 is in a bed-ridden state if an object exists above the bed-ridden state detection plane Pd and does not exist above the sitting detection plane Pr in the region of the bed 30. Further, it is determined that the object person 40 sits with the upper body thereof bent forward if an object exists above the sitting detection plane Pr and does not exist above the stand-up detection plane Ps. Since the sitting detection plane Pr is set up above the bed surface, if the sitting detection plane Pr is set up to be suitable, it rarely occurs that tossing and turning of the body or movement of a blanket is misdetected as the sitting state. In addition, when an object exists above the stand-up detection plane Ps, it is determine that the object person 40 stands up on the bed 30.

As described above, the person recognition unit 22 detects a state of the object person 40 using the detection planes, which are set up based on the bed surface Pb. The processing has advantages in that the processing is simple, for example, as compared with a case using a difference image with an image in which the object person 40 is not on the bed 30 and it does not require effort to take a background image. In addition, since an object such as a blanket is detected as a difference when using the difference image, it is difficult to distinguish the object from the object person 40. However, such a problem does not occur if a state of the object person 40 is determined using the detection planes.

In the aforementioned example, although the three steps of the detection planes are set up above the bed surface Pb in order to detect the three states in distinction from each other, it is not essential to set up the detection planes in the three steps. The detection planes may be set up in four or more steps as well as one or two steps. For example, when a state of the start of the system's operation is a bed-ridden state, the bed-ridden state may not be necessarily detected. That is, a state other than the bed-ridden state, i.e., a sitting state or a stand-up state may be detected. In such a case, two steps of the detection planes may be set up.

Further, in the aforementioned operation, although only the height location of an object with respect to the detection plane is used, a size of the object may also be determined using an area cut by the detection plane. For example, in the bed-ridden state, an area cut by the bed-ridden state detection plane Pd in a Z image is larger than that cut by the sitting detection plane Pr or the stand-up detection plane Ps. Further, in the sitting state as compared with the bed-ridden state, an area cut by the bed-ridden state detection plane Pd in the Z image is small and an area cut by the sitting detection plane Pr is large. Using such an area relationship, it is possible to accurately grasp a state of the object person 40.

For example, if in the range of the bed 30, a ratio of an area cut by the sitting detection plane Pr to an area cut by the bed-ridden state detection plane Pd is greater than or equal to a predetermined threshold value, it may be determined as a sitting state. If the threshold value is set up to be suitable, even when the object person 40 makes a long arm and stretches himself or herself so that the arm crosses the sitting detection plane Pr, since the ratio is smaller than the threshold value, it is possible to prevent such a state to be detected as the sitting state.

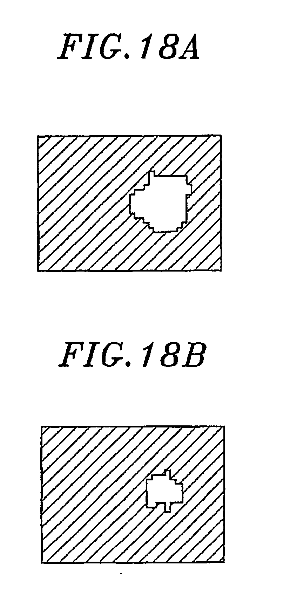

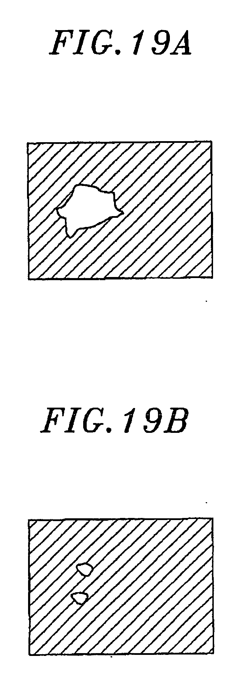

For example, each white region in Figs. 18A and 19A represents a region where the object person 40 exists in the bed-ridden state detection plane Pd, and each white region in Figs. 18B and 19B represents a region where the object person 40 exists in the sitting detection plane Pr. In Figs. 18 and 19, a ratio E2/E1 is obtained, wherein an area of the white region in Figs. 18A and 19A is E1 and an area of the white region in Figs. 18B and 19B is E2, and if the ratio E2/E1 is compared with a suitable threshold value, the sitting state (Figs. 18A and 18B) and the other state (Figs. 19A and 19B) can be distinguished from each other.

In the same manner, if in the range of the bed 30, a ratio of an area cut by the stand-up detection plane Ps to an area cut by the bed-ridden state detection plane Pd is greater than or equal to a predetermined threshold value, it may be determined as a stand-up state in which the object person 40 stands up on the bed 30. Even though there is a portion exceeding the stand-up detection plane Ps in a sitting state of the object person 40, since it is considered that an area crossing the stand-up detection plane Ps is small as compared with the stand-up state, it is possible to distinguish the stand-up state and the sitting state from each other.

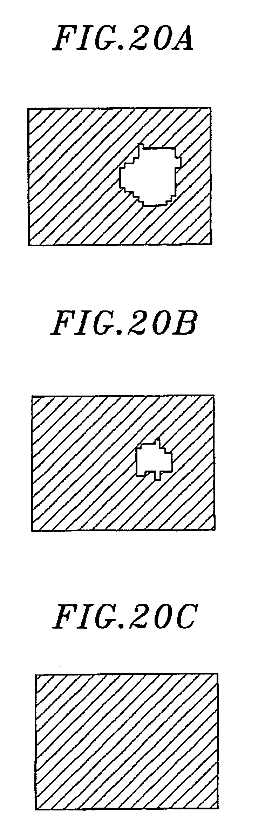

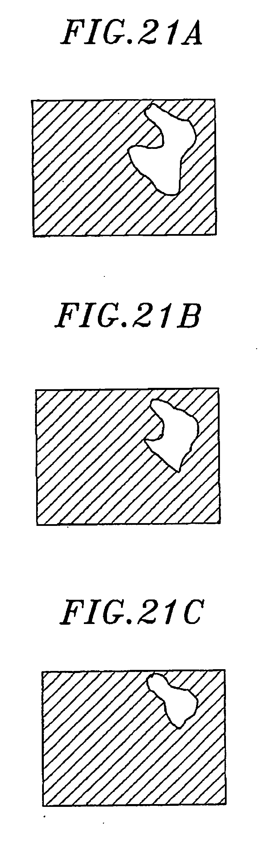

For example, each white region in Figs. 20A and 21A represents a region where the object person 40 exists in the bed-ridden state detection plane Pd, and each white region in Figs. 20C and 21C represents a region where the object person 40 exists in the stand-up detection plane Ps. Further, in the illustrated example, no white region is in Fig. 20C and the object person 40 does not exist. In addition, each white region in Figs. 20B and 21B represents a region where the object person 40 exists in the sitting detection plane Pr. Here, in Figs. 20 and 21, a ratio E3/E1 is obtained, wherein an area of the white region in Figs. 20A and 21A is E1 and an area of the white region in Figs. 20C and 21C is E3, and if the ratio E3/E1 is compared with a suitable threshold value, the sitting state (Figs. 20A to 20C) and the stand-up state (Figs. 21A to 21C) can be distinguished from each other.

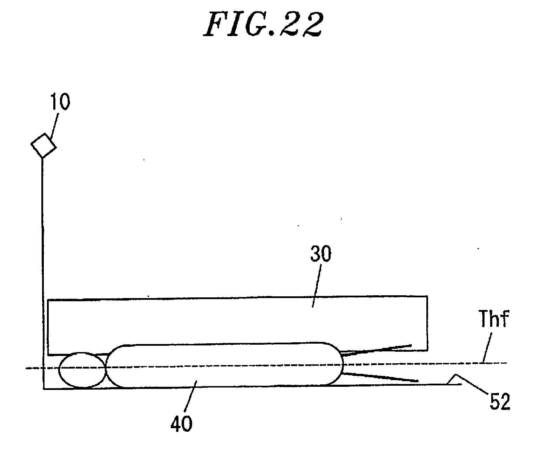

However, a state where a person is outside of the range of the bed 30 rather than a state where the person is inside of the range of the bed 30 can be detected using the height of the floor surface as a reference. That is, as shown in Fig. 22, a fallen threshold value Thf is set up by a height from the floor 52, and when the pixel outside of the range of the bed 30 in the Z image has a Z value larger than the fallen threshold value Thf, it can be determined that at least a portion of the object person 40 is outside of the range of the bed 30. The fallen threshold value Thf is set up as a value higher than the floor 52 but lower than the bed surface Pb. For example, the fallen threshold value Thf may be set up 10 cm from the floor 52.

In the aforementioned processing, although the portion outside of the range of the bed 30 is determined only by the fallen threshold value Thf, a difference image is used together outside of the range of the bed 30, whereby the object person 40 outside of the range of the bed 30 may be detected. Here, the Z image capable of being obtained from the range image taken when the system's operation starts is memorized as a background image, and a difference image obtained by subtracting a Z image capable of being obtained later from the background image is binarized.

When the system's operation starts, since the object person 40 is in the bed-ridden state, he or she does not exist outside of the range of the bed 30. Therefore, no object exists outside of the range of the bed 30 in the background image. On the other hand, an object exists outside of the range of the bed 30 in an image obtained by subtracting a Z image in which the object person 40 is outside of the range of the bed 30 from the background image. Thus, an area (the number of pixels) of a region changed in the region outside of the range of the bed 30 in the image obtained by binarizing the difference image is compared with a predefined threshold value. If the area is greater than or equal to the threshold value, it is determined that the object person 40 is outside of the range of the bed 30. Since a value outside of the range of the bed 30 in the difference image substantially corresponds to the height from the floor 52, the fallen threshold value Thf may be used as the binarized threshold value.

In addition, when the start of the system's operation is instructed, it is considered that a nurse or caregiver instructing the start of the operation exists in a region near the headboard. However, this region is outside of the region, in which the object person 40 is detected, and is also removed along with the floor 52 when the difference image is binarized. Therefore, there is no problem even if the background image is included.

As described above, the bed recognition unit 21 recognizes the location of the bed 30, and the person recognition unit 22 detects a posture of the object person 40 with respect to the bed 30. That is, since the bed 30 and the object person 40 are separated from each other, the movement determination unit 23 then classifies the movement of the object person 40.

The movement to be classified includes a state where the object person 40 perches on an end of the bed 30 (hereinafter, referred to as a "perching state"). The "perching state" is detected as a state where the object person 40 exists to span a region inside of the range of the bed 30 and a region outside thereof on any one of the four sides of the bed 30. The bed recognition unit 21 may allow the region inside of the range of the bed 30 and the region outside thereof to be divided from each other, and the person recognition unit 22 may allow the existence of the person to be detected in the region inside of the range of the bed 30 or the region outside thereof.

Using the foregoing, when person's existence regions inside and outside of the range of the bed 30 are continuous or adjacent, it is determined that the same person "crosses" the end of the bed 30. In addition, it is determined as a "perching state." Here, the term "adjacent" means that a distance between profile lines of the person's existence regions inside and outside of the range of the bed 30 is obtained and the obtained distance is within a predefined range. The distance between the profile lines may use an average value obtained on the straight lines in a direction perpendicular to the associated side of the bed 30.

In addition, a ratio of an area of the person occupied by the region outside of the range of the bed 30 to the total area of the person occupied by the regions inside and outside of the range of the bed 30 is obtained. After a suitable reference range for this ratio is set up, if this ratio is within the reference range, it may be determined as a "perching state." In such a case, it is possible to control the reference range for the area ratio in order to increase the determination accuracy of a "perching state" depending on the body type of the object person 40 or the like.

Further, it is possible to determine a "perching state" by a combination of both the aforementioned determinations. That is, if the area ratio is determined in the cross state, the possibility that a person detected inside and outside of the range of the bed 30 is the object person 40 is increased and the determination reliability is also improved.

However, the bed 30 is sometimes provided with fences for preventing a fall. In particular, it is necessary to provide fences for an object person 40 who has high possibility of falling from the bed 30. Even when such fences are provided, a doorway having no fence is provided in at least one place of the bed 30 in order to enter and leave the bed 30.

When the fences are provided as described above, a location of a doorway in the Z image is recorded in advance by an input device not shown. Since the installation location of the fence to the bed 30 is mainly selected from multiple locations (for example, six locations), a constitution of selecting the installation location of the fence may be employed using a dip switch or the like as the input device.

When the aforementioned "crossing" is detected at a region other than the doorway recorded by the input device, it is determined as a state in which a person climbs over a fence (hereinafter, referred to as a "climbing-over state").

Like the aforementioned "perching state," areas of the regions inside and outside of the range of the bed 30 may be used to determine the "climbing-over state." That is, a ratio of an area of the person occupied by the region outside of the range of the bed 30 to the total area of the person occupied by the regions inside and outside of the range of the bed 30 is obtained. After a suitable reference range for this ratio is set up, if this ratio exceeds the reference range, it may be determined as a "climbing-over state." In such a case, if the reference range is set up to be suitable, it is possible to distinguish the "climbing-over state" from a state in which an arm is swung outside of a fence.

The movement determination unit 23 may detect the movement when the object person 40 lifts his or her waist from the bed 30 and is separated from the bed 30, as an initial movement of a "leaving-bed state" (hereinafter, referred to as an "initial leaving-bed state"). In such a case, areas of the person occupied by the regions inside and outside of the range of the bed 30 are used, and the sum of the areas of the person occupied by the regions inside and outside of the range of the bed 30 is the total area of the person. After a ratio of the area of the person outside of the range of the bed 30 to the total area of the person is obtained, if this ratio approaches one (that is, if the person is not detected inside of the range of the bed 30 or the detected portion thereof is very small), it is determined as an "initial leaving-bed state." In other words, with a reference range set up to approach one, when this ratio exceeds the reference range, it is determined as the "initial leaving-bed state."

By detecting the "initial leaving-bed state," it is possible to detect an initial state of leaving a bed without tracing the object person 40. The trace is sometimes lost on its way due to any cause if using a technique of tracing the object person 40, but the aforementioned technique makes it possible to detect an "initial leaving-bed state" regardless of the passage of time.

However, when the object person 40 falls from the bed 30 or the object person 40 turns over to leave the bed 30, it is necessary to immediately provide a notification. Hereinafter, such states are collectively referred to as a "turnover state." When the "turnover state" is detected, the movement determination unit 23 measures a time during which the state continues. That is, when an object having a height less than or equal to a predefined height (for example, 30 cm) from the floor 52 is detected only outside of the range of the bed 30, it is determined that the corresponding object is a person. When a state in which a person is detected continues during a predefined determination time, the movement determination unit 23 determines that it is in a "turnover state." The determination time may be set up, for example, to be about onds.

Although each of the aforementioned "perching state," "climbing-over state," "initial leaving-bed state," and "turnover state" is determined as a specific state regardless of the passage of time, if a history of the movement of the object person 40 is traced, it is possible to more increase the determination accuracy. That is, the movement determination unit 23 traces a location of a representative point (for example, center) of the region occupied by the object person 40 detected in the person recognition unit 22.

Here, in the person recognition unit 22, a bed-ridden state, a sitting state, and a stand-up state of the object person 40 in the range of the bed 30 are respectively determined using the detection planes, and the existence placed outside of the range of the bed 30 is also determined using the fallen threshold value Thf corresponding to the detection plane. Thus, it is possible to obtain the centers of the existence regions of the object person 40 inside and outside of the range of the bed 30, which are obtained by being cut by the respective detection planes. However, the region occupied by the object person 40 may be detected in a three-dimension to obtain its center. Alternatively, mode values of X and Y values inside and outside of the range of the bed 30 may be used as X and Y values of the traced representative points.

In a case where the regions of a person inside and outside of the range of the bed 30 are combined to determine the location of the person, if a moving distance of the representative point within a predefined time is compared with a suitable threshold value, it is possible to determine whether or not a move of the representative point continuously occurs. Thus, if the move is not continuous, it can be excluded as noise. In this way, if the location of the representative point of the object person 40 is traced, it is possible to exclude noise other than the move of the object person 40, such as a move of a body part of the object person 40 or a move of bedding.

Also, since a moving path of the representative point of the object person 40 has a starting point inside of the range of the bed 30, when the representative point moves from a state in which it exists inside of the range of the bed 30 to an end of the bed 30 and follows a moving path outside of the range of the bed 30, it may be determined as a leaving-bed state. By adding such a condition, the moving path having the starting point outside of the range of the bed 30 can be determined as one caused by a person other than the object person 40 (for example, a nurse, a caregiver, a visitor, or the like). That is, when the moving path having the starting point outside of the range of the bed 30 is traced, the movement determination unit 23 does not determine that it is in a leaving-bed state.

However, in the movement of the object person 40 returning to the bed 30 after leaving the bed, it is necessary to detect the object person 40 who has returned to the bed 30. Here, after the representative point reaches a region inside of the range of the bed 30 in the moving path having the starting point outside of the range of the bed 30, if a state in which the object person 40 exists inside of the range of the bed 30 or a "perching state" continues during a predefined time, it is determined that the object person 40 has returned to the bed 30. After this determination, the starting point when the moving path of the object person 40 is traced is updated in the range of the bed 30. That is, if the object person 40 has returned to the bed 30, the starting point can be automatically returned to the initial state.

However, the technique of automatically detect whether an object is to be detected or excluded from the detection as described above may be unnecessary depending on an environment in which the device is used or the object person 40. In preparation for such a case, it is preferable to make it possible to select whether or not to perform the automatic determination of whether an object is to be detected or excluded from the detection. In addition, when an object excluded from the detection is traced, it is not that the object is automatically decided to be excluded from the detection and then the notification is not performed; but that if the device notifies the confirmation and then the notification is not released within a predetermined time, a leaving-bed state may be notified to another device.

The notification unit 24 performs a notification according to the movement of the object person 40 detected by the behavior monitoring processing unit 20. Here, the notification unit 24 has a function of setting up how a certain movement of the object person 40 is notified in conformity to the object person 40. For example, if the object person 40 is a patient or an aged person, even a sitting state or a perching state should sometimes be notified to a nurse or caregiver.

In this embodiment, all sorts of the movements of the object person 40 can be determined only by taking an image of the object person 40 using the range imaging sensor 10. In addition, which of the movements is notified may also be arbitrarily set up. The notification unit 24 is provided with a dip switch or the like, so that which of the movements is notified may be selected by providing.

Although the notification unit 24 may be disposed in the room having the bed 30 provided therein, it is preferred to notify a nurse center. For example, if the notification unit 24 has a function of generating a contact output separately from a nurse call button, it is possible to notify a leaving-bed state without pressing the nurse call button or even for a patient who cannot press it.

The notification unit 24 may be operated in relation to an operating device such as a nurse call button. That is, the notification unit 24 may be allowed to receive an input from the operating device operated by a person in addition to a determination result of the movement determination unit 23. In such a case, the determination result by the movement determination unit 23 and the input by the operating device are combined to switch between existence and non-existence of the notification of the determination result of the movement determination unit 23.

For example, for an object person 40 who requires a still state such as an absolute rest, the notification unit 24 may select the operation of notifying a nurse center when the movement determination unit 23 detects a move of the object person 40 or when a nurse call button is pressed. In addition, for the object person 40 who is allowed to move, when the movement determination unit 23 detects a move of the object person 40, a nurse center is not notified and a temporary notification state may be selected. In such a case, the notification unit 24 operates so that it releases the temporary notification state and returns to its original state when a nurse call button is pressed within a predetermined time in the temporary notification state and notifies the nurse center when the nurse call button is not pressed within the predetermined time in the temporary notification state.

As described above, the notification unit 24 can provide a preferred notification according to the movement of the object person 40 using the determination result based on the range image together with the input from the operating device such as the nurse call button. Here, the operating device is not limited to the nurse call button, but may be a touch panel of a bedside terminal or the like as described later.

Further, if the notification unit 24 is provided with a communication function using a local area network (LAN) or a nurse call communication network installed in a building and a determination result of the behavior monitoring processing unit 20 is notified to the nurse center, it is possible to remotely monitor the movement of the object person 40. In such a case, after all sorts of the movements of the object person 40 are output as status signals, a device to be notified (e.g., a main body of the nurse call or the like) may screen them to notify only desired movement. Further, movement state information of the object person 40 is superimposed on a radio transmitter for a information terminal or a PHS private branch exchange interworked with a main body of the nurse call, so that the movement of the object person 40 is grasped using PHS, a pager or the like even when a nurse or caregiver does not exist in the nurse center, and it is possible to take a rapid action.

As described above, the range imaging sensor 10 employed in this embodiment projects intensity-modulated light and simultaneously generates a range image using the quantity of received light when the intensity-modulated light is received at a light reception timing synchronized with a modulation signal. Thus, it is possible to obtain a gray scale image if averaging the quantity of received light or using the quantity of received light of a specific light reception timing.

Further, the duration, in which the light emitting elements 13 project intensity-modulated light and the imaging element 15 takes an image, and the duration, in which the light emitting elements 13 project no intensity-modulated light and the imaging element 15 takes an image, are provided, so that it may be possible to obtain a gray scale image with environment light components removed using the quantities of reception light of both the durations. The gray scale image with environment light components removed allows only an image of an object irradiated with intensity-modulated light to be taken. Further, using near-infrared light as the intensity-modulated light, it is possible to monitor the object person 40 even at night without disturbing his or her sleep.

Therefore, if it is necessary to confirm the movement determined by the behavior monitoring processing unit 20 with the naked eye, a function of switching the gray scale images may be provided. Here, the gray scale images may be displayed after the movement determined by the behavior monitoring processing unit 20 is notified. However, if the notification unit 24 has a function of memorizing the gray scale images every predetermined time, the gray scale images may be displayed back to a notification time. If such a function is provided, since the movement can be confirmed by a nurse or caregiver with the naked eye as well as the movement is notified, it is possible to take a more suitable action.

However, this function is necessary for nursing or care and may also be used when consent of the object person 40 or his or her family can be obtained. Further, when the problem is that his or her face is shown, a function of detecting a face portion is provided and then the face may be prevented from being shown by masking the face portion by digital processing. In such operation, the gray scale image may be replaced with the range image. In addition, both the range image and the gray scale image may be used. That is, the memorization or transmission of the gray scale image which will be described later can be performed for at least one of the range image and the gray scale image. Since the range image has a sufficient amount of information for grasping the movement of the object person 40 but has a small amount of information for specifying an individual, it is superior to the gray scale image in privacy protection.

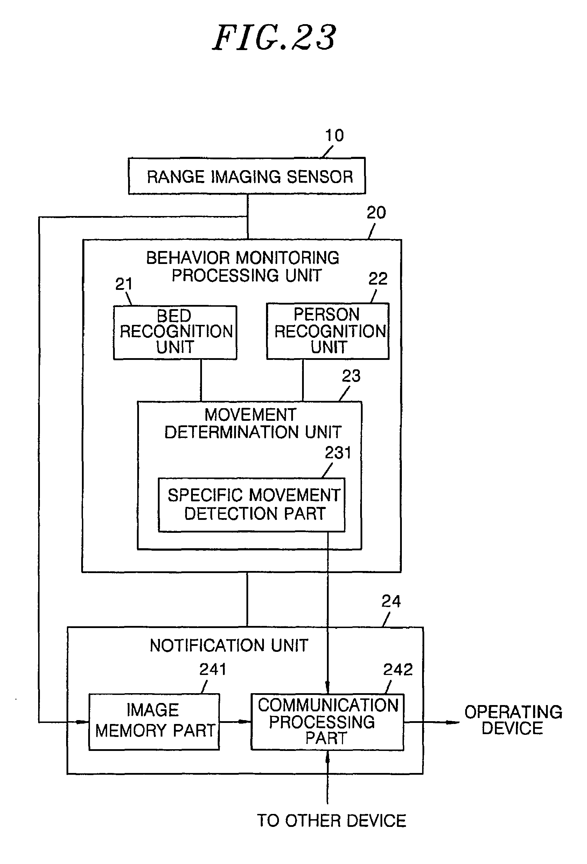

The operation when a gray scale image is memorized will be described in detail. As shown in Fig. 23, the notification unit 24 includes an image memory part 241 for memorizing gray scale images, and a communication processing part 242 for transmitting the images to another device. The image memory part 241 memorizes gray scale images for an updated predetermined time (for example, 30 seconds) output from the range imaging sensor 10 as a moving image (for example, 30 frames/second). That is, the image memory part 241 has a function of successively updating the gray scale images to be memorized like a ring buffer. In addition, the communication processing part 242 selectively transmits the gray scale image memorized in the image memory part 241 and the gray scale image output from the range imaging sensor 10 to the other device. Here, the other device includes a reception device provided in the nurse center, or the like.

The timing, at which the communication processing part 242 transmits the gray scale image to the nurse center, is determined based on an output of the movement determination unit 23. The movement determination unit 23 has a specific movement detection part 231 for detecting specific movement of a person, wherein the movement detected by the specific movement detection part 231 includes leaving-bed, turnover, or the like. If the specific movement detection part 231 detects specific movement based on the range image, the notification unit 24 reads gray scale images from the image memory part 241 for a predetermined duration before and after the movement is detected (for example, one minute total time, 30 seconds each before and after) and allows the communication processing part 242 to transmit them to the other device. Here, the notification unit 24 may also transmit gray scale images output from the range imaging sensor 10 to the other device, if necessary.

Such operation may allow the movement before and after the specific movement of the object person 40 to be confirmed in the other device such as a reception device provided in the nurse center. That is, it is possible to confirm the specific movement of the object person 40 even without going to a hospital room.

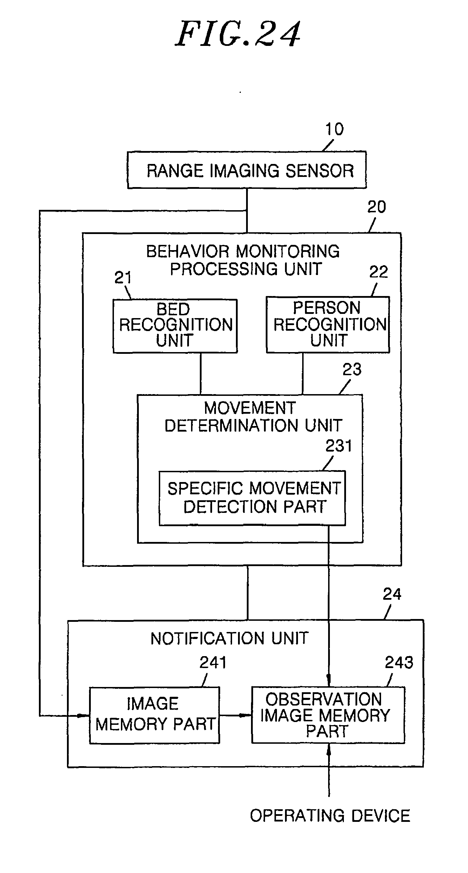

Although in the aforementioned operation, the notification unit 24 has the communication processing part 242 for transmitting gray scale images to the other device, it may have an observation image memory part 243 for memorizing gray scale images for a predetermined duration, instead of the communication processing part 242 as shown in Fig. 24. The observation image memory part 243 memorizes gray scale images for a predetermined duration before and after a detection time at which the specific movement detection part 231 detects predetermined specific movement. The gray scale images memorized in the observation image memory part 243 include gray scale images memorized in the image memory part 241 and gray scale images output from the range imaging sensor 10. That is, among the gray scale images memorized in the observation image memory part 243, the gray scale images before the movement is detected are transmitted to the image memory part 241, so that the gray scale images output from the range imaging sensor 10 are used as the gray scale images after the movement is detected.

As described above, since the observation image memory part 243 stores the gray scale images for a predetermined duration before and after the specific movement of the object person 40, behavior before and after the movement of the object person 40 can be confirmed after the fact. In such a configuration, since the notification unit 24 memorizes the gray scale images and does not transmit the gray scale images related to privacy of individuals to the other device, it is easy to secure safety. In order to increase the safety, the gray scale images memorized in the observation image memory part 243 may be encrypted.

It is preferred that the gray scale images are memorized in the observation image memory part 243 according to memorization date and time. Also, the observation image memory part 243 includes a mass storage device, such as a flash memory or a hard disk drive and is preferably a portable type so as to be detachable from the device. In such a case, the observation image memory part 243 is separated from the device and taken up, and relationships between specific movements of object persons 40 and behaviors before and after the corresponding movements can be collectively analyzed.

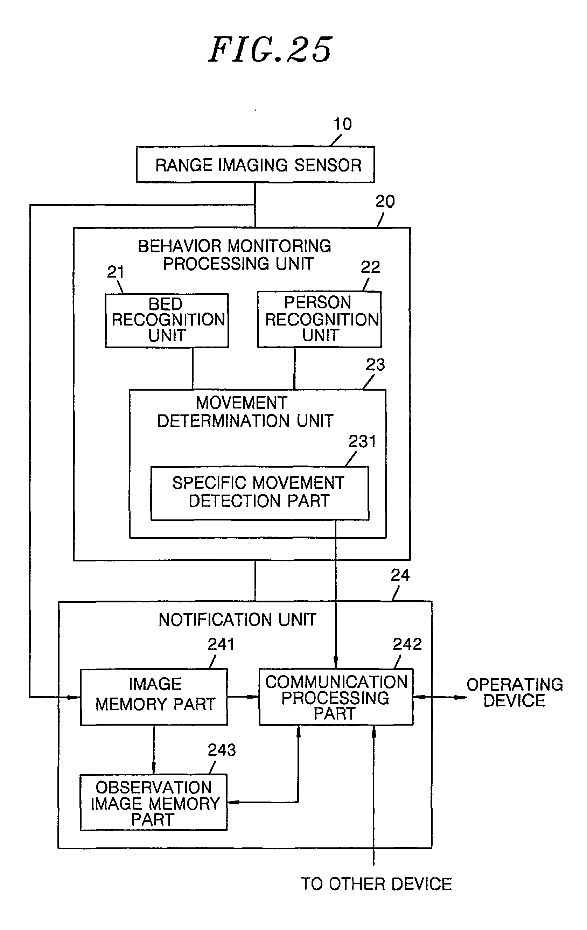

In addition, as shown in Fig. 25, the notification unit 24 may be provided with the image memory part 241, the communication processing part 242, and the observation image memory part 243, which are described above. In a case where such a configuration is employed, when the specific movement detection part 231 detects specific movement, the gray scale images for a predetermined duration before and after the detection time are not immediately transmitted to the other device but first memorized in the observation image memory part 243. At this time, the communication processing part 242 notifies the other device only that the specific movement detection part 231 detects the specified movement.

In this operation, if the transmission of the gray scale images is required by the other device, the gray scale images memorized in the observation image memory part 243 is read, and the gray scale images are transmitted to the other device through the communication processing part 242. In addition, the communication processing part 242 may be configured so that it does not transmit the gray scale image. In the configuration in which the notification unit 24 reads the gray scale image from the observation image memory part 243 for the demand from the other device and the read gray scale images are transmitted to the other device, communication traffic is reduced as compared with a case where all the gray scale images are transmitted to the other device. That is, since only the necessary gray scale images are appointed by the other device and transmitted from the notification unit 24, communication traffic is reduced and the amount of the gray scale images processed in the other device is also reduced. In addition, the gray scale images are distributed and memorized in the observation image memory part 243 of the monitoring device provided in each bed 30, so that the other device need not accumulate the gray scale images for multiple units of the beds 30 and thus the other device does not need a large memory capacity.

In addition, if the communication processing part 242 has only a function of notifying the other device that the specific movement is detected and the gray scale images memorized in the observation image memory part 243 are not transmitted to the other device, the safety of the gray scale images, which has a possibility of including personal information, is easily secured. Even in such a case, in order to increase the safety, the gray scale images may be encrypted.

Although the operation using the gray scale images has been described as an example, as described above, the memorization or transmission of the gray scale images can be performed for at least ones of the range images and the gray scale images.

However, the bed recognition unit 21, the person recognition unit 22, the movement determination unit 23, and the notification unit 24 may be implemented by executing a program in a computer. The computer may include a computer for a hospital room, which is known as a bedside terminal, in addition to a general-purpose computer. The bedside terminal is provided with a touch panel and connected through a LAN to a medical information server for centralizedly managing medical information such as electronic karte display, or administration order. In addition, the bedside terminal functions as a terminal of a wide area communication network such as the Internet, and has a function of watching a television broadcast, or the like.

Thus, the aforementioned functions are provided by executing the program in a general-purpose computer or an existing computer without using a dedicated device. In such a case, an increase in cost is also suppressed as compared with a dedicated device. When such a kind of computer is used, a communication specification such as IEEE 802.3 may be used in communications with the other device, and a communication specification such as USB (Universal Serial Bus) or IEEE 802.3 may be used in a connection with the range imaging sensor 10.

As described above, the notification unit 24 may employ a configuration of switching between existence and non-existence of the notification using the input from the operating device as well as the determination result of the movement determination unit 23. Therefore, as described above, a keyboard, touch panel, or the like may be used as the operating device when the program is executed in a computer.

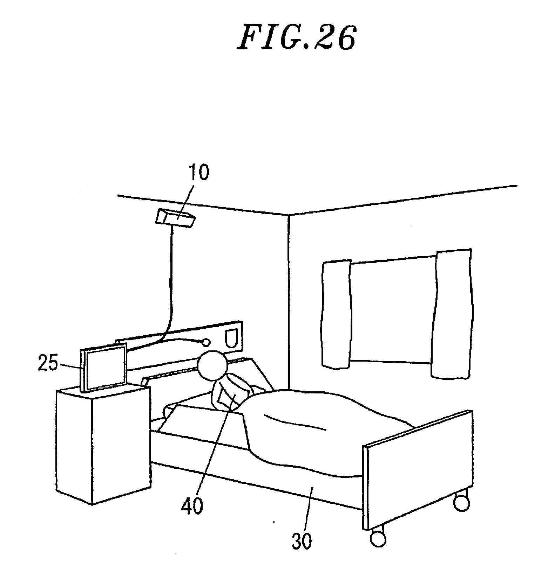

Now, suppose a bedside terminal 25 having a touch panel is used as shown in Fig. 26. When the object person 40 is prohibited from moving from the bed 30, the notification unit 24 may notify the nurse center when the movement determination unit 23 detects a move of the object person 40, or when the touch panel of the bedside terminal 25 is touched.

In addition, when the object person 40 is allowed to move from the bed 30, the notification unit 24 is in a temporary notification state when the movement determination unit 23 detects a move of the object person 40. Thereafter, if the touch panel of the bedside terminal 25 is touched within a predetermined time, the temporary notification state is released. In such a case, if the touch panel is not touched within the predetermined time after the temporary notification state, the notification unit 24 notifies the nurse center. That is, it is determined that the object person 40 sits on the bed 30 and is before leaving the bed, and thus, the nurse center is notified. Also, the behavior that the touch panel of the bedside terminal 25 is touched in the temporary notification state includes watching television, medical treatment guide, or the like through the bedside terminal if the object person 40 does not release the temporary notification state intentionally. In such a case, the notification to the nurse center is not performed and the temporary notification state is released.

In addition, when the touch panel is touched, the determination of releasing the temporary notification state may be performed in connection with the operation of the touch panel. In this operation, the temporary notification state may be correctly released by determining whether or not the touch is to intentionally operate another function or to meaninglessly support the operating unit by the physical contact in a leaving-bed behavior.

While the invention has been shown and described with respect to the embodiments, it will be understood by those skilled in the art that various changes and modification may be made without departing from the scope of the invention as defined in the following claims.

**Claims**

1.   A monitoring device, comprising:

a range imaging sensor for generating a range image wherein pixel values are distance values to an object, the entirety of a bed to be monitored being included in a field of vision of the range imaging sensor;
a bed recognition unit for extracting a location of the bed using the range image;

a person recognition unit for detecting regions occupied by a person inside and outside of the range of the bed recognized by the bed recognition unit in the range image;

a movement determination unit for determining the movement of the person relative to the bed using a combination of the regions of the bed detected by the bed recognition unit and the person detected by the person recognition unit; and

a notification unit for notifying a determination result of the movement determination unit.

2. The monitoring device of claim 1, wherein the bed recognition unit obtains frequency distributions of heights greater than or equal to a specified height from a floor surface, on which the bed is placed, in lengthwise and widthwise directions of the bed, and locations in which a frequency crosses a threshold are detected as locations of ends of the bed in the lengthwise and widthwise directions.

3. The monitoring device of claim 2, wherein the bed recognition unit set up a rectangular range surrounded by the ends of the bed in the lengthwise and widthwise directions as the range of the bed, segments the range of the bed into segmented regions in the lengthwise direction of the bed, obtains representative values of heights for respective segmented regions, and uses values obtained by subtracting a predetermined offset value from the representative values as reference heights for the respective segmented regions of the bed.

4. The monitoring device of claim 3, wherein the bed recognition unit uses one of an average value and a mode value of the heights for the segmented regions as the representative value of each of the segmented regions.

5. The monitoring device of claim 3 or 4, wherein the bed recognition unit provides an upper limit for the representative values of the segmented region and does not employ the representative values exceeding the upper limit.

6. The monitoring device of claim 3 or 4, wherein the bed recognition unit provides a threshold height value for the representative values of the segmented regions and determines that the location of the bed is abnormal when representative values of a predetermined number or more of the segmented regions exceed the threshold height value.

7. The monitoring device of claim 3 or 4, wherein immediately after the location of the bed is extracted, the bed recognition unit memorizes an existence range of an object located in the range of the bed and above a predefined exclusion threshold value for the reference heights of the segmented regions as a mask region excluded from the region of the person detected by the person recognition unit.

8. The monitoring device of any one of claims 3 to 7, wherein the person recognition unit sets up at least one detection plane spaced by a predetermined distance upwardly from the reference heights of the respective segmented regions detected by the bed recognition unit, and determines that the movement of the person is in the range of the bed defined for the detection plane if the object crossing the detection plane is detected.

9. The monitoring device of any one of claims 3 to 8, wherein the person recognition unit sets up a fallen threshold value of a predetermined height from the floor, on which the bed is placed, outside of the range of the bed detected by the bed recognition unit, and determines that the person exists outside of the range of the bed if the object is detected in a location higher than the fallen threshold value outside of the range of the bed.

10. The monitoring device of any one of claims 3 to 8, wherein the person recognition unit memorizes a background image placed outside of the range of the bed detected by the bed recognition unit, and determines that the person exists outside of the range of the bed when an area of a region in which the background image is changed in an image obtained after the memorization is greater than or equal to a predefined threshold value.

11. The monitoring device of claim 8, wherein the detection plane comprises a bed-ridden state detection plane for detecting a bed-ridden state of the person on the bed and a sitting detection plane for detecting a sitting state of the person on the bed, and the person recognition unit calculates a ratio of an area where the person exists in the sitting detection plane to an area where the person exists in the bed-ridden state detection plane when the person crosses the bed-ridden state detection plane and the sitting detection plane and determines that the person is in a sitting state if the ratio is greater than or equal to a predetermined threshold value.

12. The monitoring device of claim 8, wherein the detection plane comprises a bed-ridden state detection plane for detecting a bed-ridden state of the person on the bed and a stand-up detection plane for detecting a stand-up state of the person on the bed, and the person recognition unit calculates a ratio of an area where the person exists in the stand-up state detection plane to an area where the person exists in the bed-ridden state detection plane when the person crosses the bed-ridden state detection plane and the stand-up state detection plane and determines that the person is in a stand-up state if the ratio is greater than or equal to a predetermined threshold value.

13. The monitoring device of any one of claims 3 to 12, wherein when a region occupied by the person crosses an end of the bed in the lengthwise direction and widthwise direction, the movement determination unit determines that the person perches on the bed.

14. The monitoring device of any one of claims 3 to 12, wherein if a region occupied by the person on an end of the bed in the lengthwise direction and widthwise direction spans regions inside and outside of the range of the bed, and a ratio of an area of the region occupied by the person outside of the range of the bed to an area of the region occupied by the person is within a predefined reference range, the movement determination unit determines that the person perches on the bed.

15. The monitoring device of any one of claims 3 to 12, wherein when the bed is provided with a fence for preventing a fall and a location of the fence can be specified in advance, if a region occupied by the person crosses the fence, the movement determination unit determines that the person climbs over the fence.

16. The monitoring device of any one of claims 3 to 12, wherein when the bed is provided with a fence for preventing a fall and a location of the fence can be specified in advance, if a region occupied by the person spans regions inside and outside of the range of the bed at the fence and a ratio of an area of a region occupied by the person outside of the range of the bed to an area of the region occupied by the person exceeds a predefined reference range, the movement determination unit determines that the person climbs over the fence.

17. The monitoring device of any one of claims 3 to 16, wherein when a ratio of an area of a region occupied by the person outside of the range of the bed to an area of the regions occupied by the person inside and outside of the range of the bed exceeds a predefined reference range, the movement determination unit determines that the person leaves the bed.

18. The monitoring device of any one of claims 3 to 17, wherein when an object having a height less than or equal to a predefined height with respect to the floor surface, on which the bed is placed, outside of the range of the bed is detected continuously for a predefined determination time, the movement determination unit determines that the person falls down.

19. The monitoring device of any one of claims 3 to 18, wherein the movement determination unit traces a location of a representative point of the person detected inside and outside of the range of the bed, and the movement determination unit does not determines that the person moves if the location moves discontinuously,.

20. The monitoring device of claim 19, wherein when the location of the representative point of the person is traced, the movement determination unit determines that the person behaves if a starting point is inside of the range of the bed, and updates the starting point of tracing inside of the range of the bed if the starting point is outside of the range of the bed and the person is inside of the range of the bed continuously for a predefined time after the person has moved into the range of the bed.

21. The monitoring device of any one of claims 1 to 20, wherein the range imaging sensor has a function of converting a coordinate system defined for the range image into a coordinate system of a space having the bed placed therein.

22. The monitoring device of any one of claims 1 to 21, wherein the notification unit has a function of allowing a notification to correspond with the movement of the person.

23. The monitoring device of any one of claims 1 to 22, wherein the notification unit is provided with a communication function of notifying a separately provided reception device of a notification.

24. The monitoring device of any one of claims 1 to 23, wherein the notification unit receives an input from an operating device operated by a person in addition to the determination result of the movement determination unit, and combines the determination result of the movement determination unit and the input by the operating device to switch between existence and non-existence of a notification of the determination result of the movement determination unit.

**25.** The monitoring device of any one of claims 1 to 24, wherein the range imaging sensor has a function of generating a gray scale image together with the range image, and the notification unit outputs at least one of the range image and the gray scale image after notifying the movement of the person.

**26.** The monitoring device of claim 25, wherein the movement determination unit has a specific movement detection part for detecting a specific movement of the person; and the notification unit has an image memory part for memorizing at least one of the gray scale image and the range image generated by the range imaging sensor, and a communication processing part for transmitting at least one of the range images and the gray scale images for a predetermined duration before and after the specific movement detection part detects the specific movement of the person from the image memory part and the range imaging sensor to another device.

**27.** The monitoring device of claim 25, wherein the movement determination unit has a specific movement detection part for detecting a specific movement of the person; and the notification unit has an image memory part for memorizing at least one of the gray scale image and the range image generated by the range imaging sensor, and an observation image memory part for memorizing at least one of the range images and the gray scale images for a predetermined duration before and after the specific movement detection part detects the specific movement of the person.

**28.** The monitoring device of claim 25, wherein the movement determination unit has a specific movement detection part for detecting a specific movement of the person; and the notification unit has an image memory part for memorizing at least one of the gray scale image and the range image generated by the range imaging sensor, an observation image memory part for memorizing at least one of the range images and the gray scale images for a predetermined duration before and after the specific movement detection part detects the specific movement of the person, and a communication processing part for notifying the other device that the specific movement detection part detects the specific movement of the person.

**29.** A program for allowing a computer to function as a bed recognition unit for extracting a location of a bed to be monitored using a range image obtained from a range imaging sensor, the range imaging sensor generating the range image wherein pixel values are distance values to an object, the entirety of the bed being included in a field of vision of the range imaging sensor; a person recognition unit for detecting regions occupied by a person inside and outside of the range of the bed recognized by the bed recognition unit in the range image; a movement determination unit for determining a movement of the person relative to the bed using a combination of the regions of the bed detected by the bed recognition unit and the person detected by the person recognition unit; and a notification unit for notifying a determination result of the movement determination unit.

## FIG.1

RANGE IMAGING SENSOR — 10

BEHAVIOR MONITORING PROCESSING UNIT — 20

BED RECOGNITION UNIT — 21

PERSON RECOGNITION UNIT — 22

MOVEMENT DETERMINATION UNIT — 23

NOTIFICATION UNIT — 24

EP 2 589 330 A1

*FIG.2A*

*FIG.2B*

25

# FIG.3

# FIG.4

## FIG.5A

## FIG.5B

## FIG.5C

*FIG.6*

## FIG.7A

FREQUENCY

Thx

Xi        Xs        X

## FIG.7B

FREQUENCY

Thy

Yi        Ys        Y

## FIG.8

Ymin

40

30

X

Xmax

Ymax

Fv

## FIG.9A

40

37    37    37    30

## FIG.9B

40    Vt    Vt

30

## FIG.10

Vh

40

Vh

Vh

30

# FIG.11

# FIG.12

## FIG.13

## FIG.14

# FIG.15A

# FIG.15B

## FIG.16A

## FIG.16B

# FIG.17

## FIG.18A

## FIG.18B

# FIG. 19A

# FIG. 19B

## FIG.20A

## FIG.20B

## FIG.20C

## FIG.21A

## FIG.21B

## FIG.21C

# FIG.22

# FIG.23

RANGE IMAGING SENSOR — 10

BEHAVIOR MONITORING PROCESSING UNIT — 20

BED RECOGNITION UNIT — 21

PERSON RECOGNITION UNIT — 22

MOVEMENT DETERMINATION UNIT — 23

SPECIFIC MOVEMENT DETECTION PART — 231

NOTIFICATION UNIT — 24

IMAGE MEMORY PART — 241

COMMUNICATION PROCESSING PART — 242

OPERATING DEVICE

TO OTHER DEVICE

# FIG.24

# FIG.25

```
                                    10
                    ┌─────────────────────────┐
                    │  RANGE IMAGING SENSOR   │
                    └─────────────────────────┘
                                              20
         ┌──────────────────────────────────────┐
         │   BEHAVIOR MONITORING                 │
         │     PROCESSING UNIT                   │
         │    21                      22         │
         │  ┌──────────┐         ┌──────────┐    │
         │  │   BED    │         │  PERSON  │    │
         │  │RECOGNITION│        │RECOGNITION│   │
         │  │   UNIT   │         │   UNIT   │    │
         │  └──────────┘         └──────────┘    │
         │                              23       │
         │  ┌─────────────────────────────────┐  │
         │  │        MOVEMENT                 │  │
         │  │   DETERMINATION UNIT            │  │
         │  │                       231       │  │
         │  │  ┌───────────────────────────┐  │  │
         │  │  │  SPECIFIC MOVEMENT        │  │  │
         │  │  │  DETECTION PART           │  │  │
         │  │  └───────────────────────────┘  │  │
         │  └─────────────────────────────────┘  │
         └──────────────────────────────────────┘
                                              24
   ┌────────────────────────────────────────────┐
   │          NOTIFICATION UNIT                  │
   │      241                       242          │
   │  ┌──────────┐         ┌─────────────┐       │      OPERATING
   │  │  IMAGE   │────────▶│COMMUNICATION│──────────────▶ DEVICE
   │  │  MEMORY  │         │ PROCESSING  │       │
   │  │  PART    │         │    PART     │       │
   │  └──────────┘         └─────────────┘       │
   │        │      243                           │
   │        ▼                                    │
   │  ┌──────────┐                               │
   │  │OBSERVATION│                              │
   │  │IMAGE MEMORY│                             │
   │  │   PART    │                              │
   │  └──────────┘                               │
   └────────────────────────────────────────────┘
                         TO OTHER DEVICE
```

# FIG.26

## INTERNATIONAL SEARCH REPORT

| International application No. |
|---|
| PCT/IB2011/001360 |

A. CLASSIFICATION OF SUBJECT MATTER
*A61B5/00*(2006.01)i, *A61B5/11*(2006.01)i

According to International Patent Classification (IPC) or to both national classification and IPC

B. FIELDS SEARCHED

Minimum documentation searched (classification system followed by classification symbols)
A61B5/00, A61B5/11

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-88512 A (Sumitomo Osaka Cement Co., Ltd.), 25 March 2003 (25.03.2003), abstract & US 2004/0210155 A1 & EP 1410755 A1 & WO 2002/102242 A1 | 1-29 |
| A | JP 8-150125 A (Kanebo, Ltd.), 11 June 1996 (11.06.1996), abstract; paragraphs [0025] to [0029] (Family: none) | 1-29 |
| P,A | JP 2011-103037 A (Toshiba Corp.), 26 May 2011 (26.05.2011), entire text; all drawings (Family: none) | 1-29 |

| ☐ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 29 August, 2011 (29.08.11) | 06 September, 2011 (06.09.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**EP 2 589 330 A1**

**REFERENCES CITED IN THE DESCRIPTION**

**Patent documents cited in the description**

*   JP 2003290154 A